# EUROPEAN PATENT APPLICATION

(11) **EP 2 562 544 A1**
(43) Date of publication of application: **27.02.2013**
(21) Application number: 12177482.2
(22) Date of filing: 20.12.2007
(51) Int. Cl.: G01N 33/68, C12Q 1/68, G01N 33/53

(54) **kits for use in the differentiation of IBD and IBS and further distinction between disease types of IBD**

(62) Divisional of application: 07861152.2
(71) Applicant: InDex Diagnostics AB, 171 77 Stockholm (SE)
(72) Inventor: Von Stein, Petra, 194 34 Upplands Väsby (SE); Kouznetsov, Nikolai, 176 72 Järfälla (SE); Von Stein, Oliver, 194 34 Upplands Väsby (SE); Gielen, Alexander, 124 76 Bandhagen (SE)
(74) Representative: Holmberg, Martin Tor

(57) **Abstract**

Diagnostic kits for use in a method for differentiation between IBD and IBS comprising at least two antibodies directed to at least two proteins or protein fragments chosen from the group comprising the following proteins (protein accession no.): SLC6A14 (NP_009162); SLC26A2 (NP_000103); GRO-1 (NP_001502); MMP-7 (NP_002414); MAP-17 (NP_005755); RegIV (NP_114433); and Vanin-1 (NP_004657).

## Description

The present invention relates to a method for use in the diagnosis of inflammatory bowel disease using a multi-gene approach. More particularly, the present invention relates to a method and kit for use in the diagnosing and/or differentiating inflammatory bowel disease (IBD) from inflammatory bowel syndrome (IBS) by determining the expression of at least one specific marker gene or genes, e.g. using oligonucleotides specifically amplifying said marker genes, or determining the expression of the corresponding proteins, e.g. using antibodies specifically recognizing the proteins encoded by said specific marker gene or genes. The diagnosing method and corresponding kit can be advantageously used in the early diagnosis and differentiation of these diseases due to the improved accuracy and reproducibility.

### Background of the invention

Inflammatory bowel disease (IBD) describes a state of chronic relapsing intestinal inflammation. The current understanding of disease pathogenesis suggests a complex interplay of multiple environmental and genetic factors. It causes significant morbidity in populations of European origin, with the two major forms - Crohn's disease (CD) and ulcerative colitis (UC) - having a combined prevalence of 150-250 / 10000. Both forms of IBD have a peak incidence in early adult life but can develop at any stage. They affect the sexes to an approximately equal extent. Environmental risk factors for the development of IBD are poorly defined. The best characterized factor is smoking, which clearly increases the risk of developing Crohn's disease but protects against ulcerative colitis, for reasons that are not clear.

Disease management is a long-term commitment for both patient and physician, since there is currently no cure for either condition. Approximately 30% of IBD patients undergo surgery during their lifetime and patients with long-standing IBD are at considerable risk of developing colorectal cancer. Three out of ten IBD patients do not respond to the best available medical therapy today, even when high doses are used, causing considerable side effects.

Ulcerative colitis typically causes symptoms of bloody diarrhea and faecal urgency. Inflammation involves the rectum, and can extend in a continuous manner to parts of, or the entire proximal colon. Histological examination reveals an infiltrate of chronic inflammatory cells, which are restricted to the superficial layers of the colonic mucosa. Granulomata are not a feature. Crohn's disease is rather more pleomorphic. It is characterized pathologically by discontinuous segments of transmural inflammation, which can affect any part of the gastrointestinal tract from mouth to anus, but most commonly involve the ileocaecal region. Granulomata are a histological hallmark but the clinical features are rather variable depending on the site of bowel involvement. If the colon is inflamed, the symptoms can exactly mimic those of ulcerative colitis.

It is obvious that a possibility to clinically distinguish UC from colonic CD at an early stage would provide enormous benefits for both the patient and the physician. It would permit the design of accurate treatment regimes, prevent the use of unnecessary medications and reduce treatment costs.

Irritable bowel syndrome, or IBS, is also a gastrointestinal disorder in which the smooth muscles of the intestinal wall become hypersensitive to triggers such as stress or even coffee and cause bloating, nausea, vomiting and constipation or diarrhoea. Unfortunately, as many of the symptoms are similar to those of IBD, IBS is often misdiagnosed.

There are some five millions cases of IBS in the USA alone. Enabling to distinguish IBS from the more serious IBD cases would reduce the number of unnecessary visits to the doctor by IBS patients who are suspected to have IBD. It would also help the doctor to choose the most appropriate type of treatment.

The patent literature indicates that the available methods for distinguishing between different forms of IBD, and in particular the differentiation between UC and CD, apart from the above given examples of different examination procedures, have been focused on antibody based methods.

For example WO 03/036262 describes a method and kit for the differentiation of CD from other gastrointestinal illnesses, such as UC and IBS, using the presence of faecal anti-*Saccharomyces cerevisiae* antibodies (ASCA) as a marker for Crohn's disease are provided. The kit includes an enzyme-linked immunoassay or other immunoassay that utilizes antibodies specific to human immunoglobins for the measurement of total endogenous ASCA in a human faecal sample.

WO01/58927 describes diagnostic methods for detecting diseases associated with an autoantigen response to hTM in affected tissue, and in particular UC.

Another intensively studied antibody approach (see U.S. patents 5,691,151; 5,830,675; and 6,033,864) are the autoantibodies to neutrophils (atypical perinuclear anti-neutrophil cytoplasmic antibodies) P-ANCAs. They are found mainly in UC (50-67%) but also in CD (6-15%). The combination of ANCAs and ASCAs was thought to be of good help in patients with indeterminate colitis. But a remarkable finding was that about 50% of the patients with indeterminate colitis are seronegative patients. This means that they do not develop antibodies for ASCAs and ANCAs and thus cannot be diagnosed for, in average, nearly 10 years (Bossuyt X., Clin Chem 2006, 52:2, 171-181)

WO 2004/001073 describes the seven genes referred in the present invention as diagnostic markers for UC. The expression of these markers on RNA level is determined in mucosal biopsies from the colon.

### Summary of the invention

There remains a need for improved methods for the accurate, rapid and reliable screening of patients reporting gastrointestinal problems, and to distinguish between IBD and non-IBD patients, e.g. patients suffering from IBS. It would be particularly desirable, both from a patient and a hospital perspective, if a first screening could be performed before the patient is remitted to ileocolonoscopic examination.

There is also a need for improved diagnosis of ulcerative colitis, in particular in the context of distinguishing between ulcerative colitis and Crohn's disease in IBD patients.

One aim of the present invention is to make available methods and kits for these purposes. One particular aim is to make available a method and kit which makes it possible to reach a reliable diagnosis at an early stage of the disease. Another aim is to make available a method for a reliable and yet simplified and less invasive method to follow the response to treatment and confirming the improvement in a patient undergoing treatment.

Another aim is to make it possible to perform a first screening using non-invasive or minimally invasive sampling techniques, in contrast to the biopsies or ileocolonoscopic examination normally used. Also the barium enema examination should be classified as an invasive procedure, due to the considerable discomfort and strain it exerts on the patient. It is also an aim to make available methods and kits for distinguishing between IBD and IBS, and CD and UC also in difficult cases, where the clinical picture can be very similar.

Further aims underlying the invention, as well as the solutions offered by the invention and the associated advantages will become evident to a skilled person upon study of the description, examples and claims.

The present inventors have surprisingly found that a differentiation between IBD and IBS, and in the case of having determined that the patient suffers from IBD, a further distinction between UC and CD, is made possible by a multi-gene approach where the gene expression profiles are studied in a sample taken from the human body, preferably taken from faeces or blood of a patient, or a biopsy sample obtained from an inflamed area in the intestines.

The present invention is based on the identification of potential marker genes which either collectively, singularly or in sub-groups, form the basis for a distinction between IBD and IBS.

One embodiment of the invention, experimentally confirmed, is that the quantification of the expression levels of a number of specific genes and the corresponding proteins can be utilized in accurately and simply determining, using samples from faeces or blood, whether the patient is suffering from IBD, and in a follow up analysis using a biopsy, determine if the patient is afflicted with UC or CD.

More specifically, methods are provided that allow the detection of seven (7) distinct markers, singularly or in sub-groups thereof, using gene specific oligonucleotides or antibodies for quantification of the expression levels of said markers. The oligonucleotides or antibodies are designed to recognize specifically the corresponding gene sequence or marker protein. An assay and kit for the detection and monitoring expression status of said seven marker genes or sub-sets thereof in a biological sample are provided. The marker genes are (Accession numbers within parenthesis):
SLC6A14 (NM_007231); SLC26A2 (NM_000112); GRO-1 (NM_001511); MMP-7 (BC003635); MAP-17 (NM_005764); RegIV (BC017089), and Vanin-1 (NM_004666).

Results obtained by the inventors indicate that Vanin-1 is a preferred marker gene when analyzing RNA in a blood sample. Also the markers SLC26A2, MAP-17 show utility in blood.

The assay is preferably a method and kit for the quantification of RNA based on PCR-methods.

Alternatively, the assay is a non-culture, antibody-based assay for the detection of said marker proteins.

Another embodiment of the present invention is thus a method for effectively detecting UC or CD in an early stage using antibodies specifically binding to proteins expressed by the human genes SLC6A14, SLC26A2, GRO-1, MMP-7, MAP-17, Reg IV and Vanin-1 in a sample taken from the human body and a kit for diagnosing UC and CD using the same. Results obtained by the inventors indicate that GRO-1 and RegIV are the preferred marker proteins when the source of the sample is human faeces, and Vanin-1, GRO-1 and MMP-7 when the sample is blood.

Another embodiment of the present invention is a method for diagnosing UC and CD by measuring the level of expression of SLC6A14, SLC26A2, GRO-1, MMP-7, MAP-17, Reg IV and Vanin-1 proteins in a sample by an antigen-antibody binding reaction using an antibody specifically binding to a protein expressed from human genes encoding SLC6A14, SLC26A2, GRO-1, MMP-7, MAP-17, Reg IV and Vanin-1, and a kit for diagnosing UC and CD using the same. Further embodiments of the invention are methods for the determination of a patient's response to a treatment, the rate of improvement during treatment and for estimating the prognosis for recovery.

### Brief Description of the Drawings

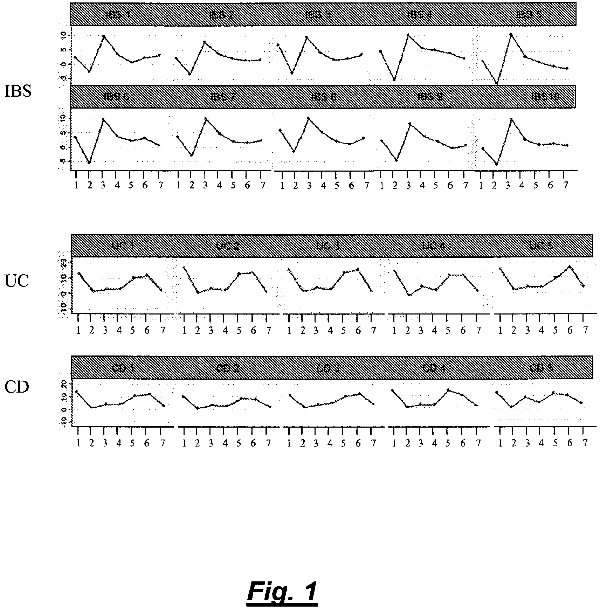

The invention will be disclosed in detail in the following description, examples and drawings, in which
Figure 1 shows expression profiles of 10 IBS and 10 IBD cases derived from real-time analysis of faeces specimen. The seven different markers are plotted on the x-axis, and the corresponding delta Ct values of the PCR analysis are plotted on the y-axis (1=SLC6A14; 2=SLC26A2; 3=GR01; 4=MMP-7; 5=MAP17; 6=GISP; 7=Vanin-1).

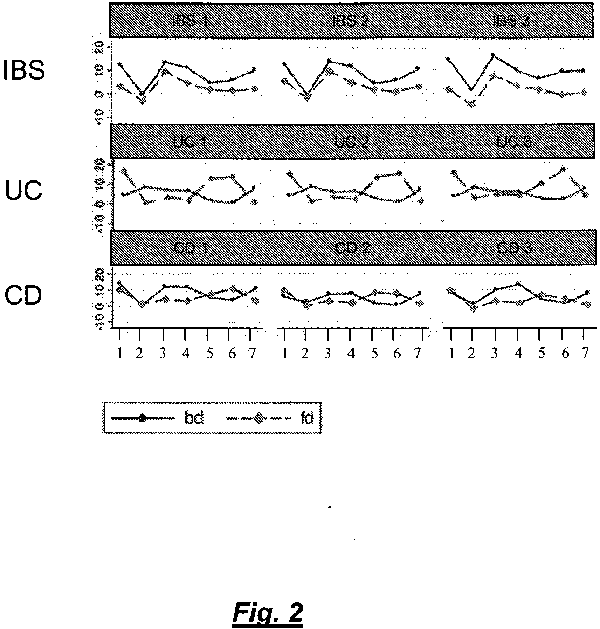

Figure 2 shows the expression profiles in faeces and biopsy samples derived from real-time PCR analysis. The seven different markers are plotted on the x-axis, and the corresponding delta Ct values of the PCR analysis are plotted on the y-axis (1=SLC6A14; 2=SLC26A2; 3=GRO1; 4=MMP-7; 5=MAP17; 6=GISP; 7=Vanin-1). [bd=biopsy derived; fd= faeces derived]

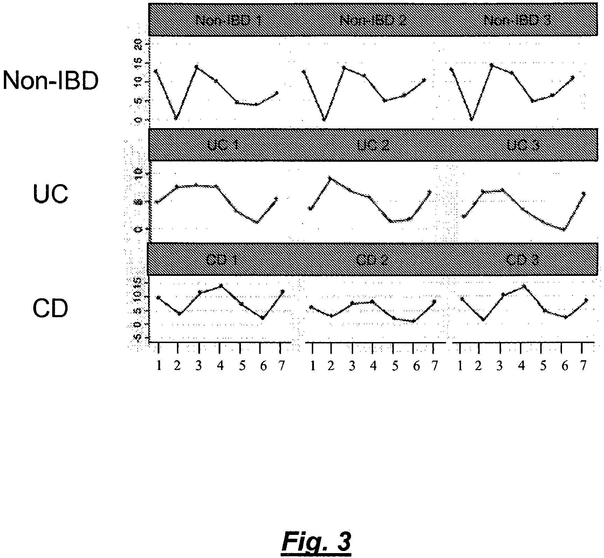

Figure 3 shows the expression profiles in biopsy samples derived from real-time analysis. The seven different markers are plotted on the x-axis, and the corresponding delta Ct values of the PCR analysis are plotted on the y-axis (1=SLC6A14; 2=SLC26A2; 3=GRO1; 4=MMP-7; 5=MAP17; 6=GISP; 7=Vanin-1).

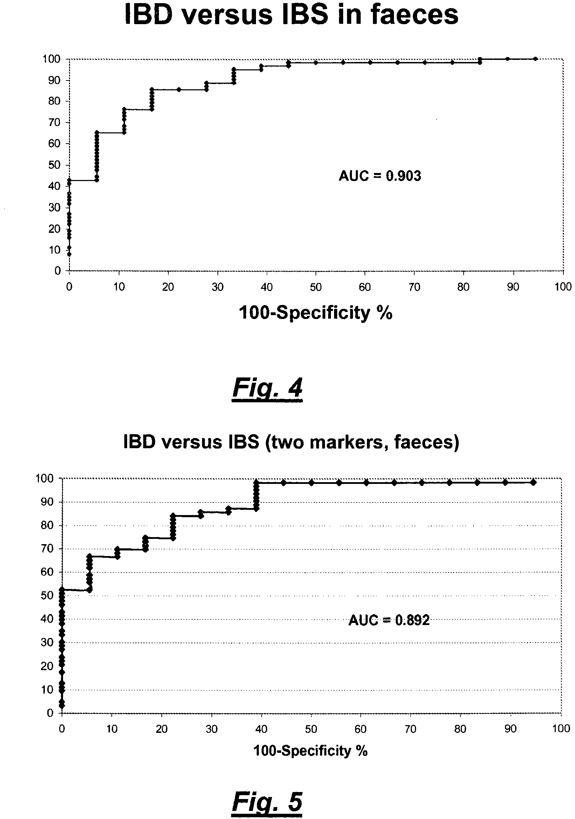

Figure 4 shows a ROC plot as a result of the real-time analysis of 18 non-IBD and 63 IBD patients with all seven markers followed by an analysis with a classification algorithm. The area under the ROC curve (AUC) of 0.903 illustrates the high discriminative potential of all the markers together.
Figure 5 shows a ROC plot as a result of the real-time analysis of 18 non-IBD and 63 IBD patients with only two markers (GRO-1 and Reg IV) followed by an analysis with a classification algorithm. The area under the ROC curve (AUC) of 0.892 illustrates the high discriminative potential of these markers alone.

### Detailed description

Before the present invention is disclosed and described, it is to be understood that one skilled in the art will appreciate readily that the present invention is well adapted to carry out the objects and obtain the ends and advantages mentioned, as well as those objects, ends and advantages inherent herein. The present examples, along with the methods, procedures, treatments, molecules, and specific compounds described herein are presently representative of preferred embodiments, are exemplary, and are not intended as limitations of the scope of the invention. Changes therein and other uses that will occur to those skilled in the art are encompassed within the spirit of the invention as defined by the scope of the claims.

It is also to be understood that the terminology used herein is for purposes of describing particular embodiments only, and is not intended to be limiting. It must be noted that, as used in the specification and the appended claims, the singular forms "a," "an" and "the" include plural referents unless the context clearly dictates otherwise. Thus, for example, reference to "a sequence" includes more than one such sequence, and the like.

In this specification and in the claims which follow, reference will be made to a number of terms which shall be defined to have the following meanings:

As used herein, the term "complementary DNA primer" means an oligonucleotide, which anneals to the RNA template in a particular orientation to allow for the synthesis of a nascent DNA strand in the presence of reverse transcriptase in the biological sample under the conditions described herein.

Also as used herein, the "condition" under which a DNA strand is synthesized include the presence of nucleotides, cations and appropriate buffering agents in amounts and at temperatures, such that the RNA template and the DNA primer will anneal and oligonucleotides will be incorporated into a synthesized DNA strand if reverse transcriptase is not inhibited by the reverse transcriptase inhibitor drug. Exemplary conditions are set forth in the examples below. The described conditions have been optimized from other known RT/cDNA synthesis protocols. It is generally known that other conditions can be established for optimization of a particular reverse transcriptase reaction on the basis of protocols well known to one of ordinary skill in the art.

As used herein, the term "primer pair" refers to two primers, one having a forward designation and the other having a reverse designation relative to their respective orientations on a double-stranded DNA molecule which consists of a sense and antisense sequence, such that under the amplification conditions described herein, the forward primer anneals to and primes amplification of the sense sequence and the reverse primer anneals to and primes amplification of the antisense sequence. Primers can be selected for use in the amplification reaction on the basis of, having minimal complementarity with other primers in the reaction (to minimize the formation of primer dimers) and having Tm values with the range of reaction temperatures appropriate for the amplification method, preferably PCR. In addition, primers can be selected to anneal with specific regions of the RNA template such that the resulting DNA amplification product ranges in size from 100 to 500 base pairs in length and most preferably around 300 base pairs in length.

As used herein, the terms "detecting" or "detection" of the amplified DNA refers to qualitatively or quantitatively determining the presence of the amplified DNA strand, which is only synthesized if reverse transcriptase is resistant to the reverse transcriptase inhibitor drug added to the assay mixture. The amplification of the synthesized DNA can be detected by any method for the detection of DNA known in the art. For example, detection of the amplified DNA can be by Southern blot hybridization assay, by visualization of DNA amplification products of specific molecular weight on ethidium bromide stained agarose gels, by measurement of the incorporation of radiolabeled nucleotides into the synthesized DNA strand by autoradiography or scintillation measurement.

In the method of the present invention, PCR amplification is accomplished by pre-incubating all PCR reagents and a sample containing a target nucleic acid in the presence of appropriate gene specific primers and a thermostable polymerase enzyme. The resulting reaction mixture is cyclically heated under conditions allowing for the formation and amplification of primer extension products.

The reagents required for PCR are known to persons skilled in the art, and generally include at least two oligonucleotide primers that are sufficiently complementary to conserved regions of the target nucleic acid to hybridise thereto, four different nucleoside triphosphates, a thermostable polymerisation agent and any requisite cofactors for the polymerisation agent. Preferred nucleoside triphosphates are the deoxyribonucleoside triphosphates dATP, dCTP, dGTP and dTTP or dUTP, collectively termed dNTPs. Nucleoside triphosphates are commercially available.

Primers include naturally occurring or synthetically produced oligonucleotides capable of annealing to the target nucleic acid and acting as the point of initiation of nucleic acid synthesis under appropriate conditions, i.e., in the presence of nucleoside triphosphates, a polymerisation agent, suitable temperature, pH and buffer. The primers have sequences sufficiently complementary to the target nucleic acid to hybridise thereto, and are of sufficient length, typically from 10-60 nucleotides, to prime the synthesis of extension products in the presence of a polymerisation agent. Primers can also be produced synthetically by automated synthesis by methods well known to one of ordinary skill in the art.

Design considerations for primers are well known in the art. Primers are selected to be substantially complementary to the sequences of the strands of the specific nucleic acid to be amplified, such that the extension product synthesized from one primer, when separated from its complement, can serve as a template for the extension product of the other primer. Preferably, the primers are exactly complementary with the target region. It is underlined that the primer pairs given in the present specification, examples and claims can be replaced by functionally equivalent primers, exhibiting specificity to the marker genes, without departing from the scope of the invention.

The present inventors collected faeces samples (stool samples) of 33 UC and 24 CD for analysis with real-time PCR to determine if they could discriminate between UC and CD as described for biopsy material. Surprisingly, the inventors found that the expression profiles of these markers were substantially the same for UC and CD in the faeces specimen. As control samples, some IBS cases were used. These exhibited a different expression pattern for the genes as the two major forms of IBD. The inventors analyzed in total 18 IBS cases against the 63 IBD cases. The results in Fig. 1 show a clear difference in the expression profile between IBS and IBD for 10 IBS and 10 IBD cases.

Another surprising fact was that genes which were up-regulated for UC in biopsies were now down-regulated in faeces samples (marker 6 and also to some extent marker 5). Also marker 2 (SLC26A2) was originally down-regulated in UC but now found to be up-regulated compared to marker 1 (SLC14A6). In Figure 2, the expression profiles of faeces and biopsy samples of 3 IBS, 3 UC and 3 CD patients are shown.

In the case of the IBS patients the expression profile of the genes does not change between the different specimens. However, when the profiles in the UC cases are compared, an opposite trend of the two curves can be observed, especially in terms of the contrary expression of markers 2, 5 and 6. Also for CD, the expression pattern is changed in the biopsies (see fig 3).

Solute carrier (SLC) proteins comprise of a very large family of energy dependent transport molecules and have critical physiological roles in nutrient transport and may be utilized as a mechanism to increase drug absorption. However, there is limited understanding of these proteins at the molecular level due to the absence of high-resolution crystal structures.

In total, 1-2% of adults and 6-8% of children suffering from kidney stones have cystinuria, a defect in the transport of amino acids, which leads to high concentrations of cystine in the urine. Two genes have been implicated, solute carrier family 3, cystine, basic and neutral amino acid transporter, member 1 (SLC3A1) coding for the protein related to the system of amino-acid transporter, and solute carrier family 7, member 9 (SLC7A9). Both of these solute carriers are believed to be involved in stone formation which may ultimately lead to urinary tract infection and, eventually, renal failure.

The inventors have now identified two known solute carriers (SLC6A14 and SLC26A2) whose expression is significantly altered in IBD.

CXC chemokine growth-related oncogene-alpha (Gro-alpha also known as GRO 1) is as described a cytokine and as such can alter the migratory responses of numerous cell types in local areas of inflammation. It has been described to be over expressed in human inflamed corneas (Spandau *et al.,* 2003) and in addition, it has also been shown that rats chemically induced to exhibit inflammation of the gut show up-regulated levels of GRO 1 (Hirata *et al.,* 2001). Using a cDNA microarray approach, Heller **et a**l., 1997 describes novel participation of the chemokine Gro alpha in rheumatoid arthritis and inflammatory bowel disease. Keiichi *et al.,* 2006 reported increased circulating concentrations of Growth-Related Oncogene (GRO)-α in patients with IBD. While it is described in Isaacs *et al.,* 1992, that expression of GRO1 in UC is higher than that seen in CD, here it has been demonstrated that there exists an inverse correlation of UC verses CD with respect to GRO 1 expression levels. Lastly Lawrence *et al.,* 2001 describes identifying GRO 1 as being up-regulated in UC, but the design of the study was such that biopsy samples where pooled before analysis, therefore is was not possible to know whether GRO 1 was up-regulated in more than 1 patient.

Matrilysin or (matrix metalloproteinase-7) was first discovered in the involuting rat uterus; it has also been known as uterine metalloproteinase, putative metalloproteinase (Pump-1), and matrix metalloproteinase 7 (MMP-7). It is the smallest member (28 kDa) of a family of 15 MMPs that together are able to degrade most of the macromolecules of the extra cellular matrix. This family is briefly reviewed; all members are zinc metalloproteinases that occur in zymogene form with the active site zinc blocked by cystine. Matrilysin can degrade a wide range of gelatins, proteoglycans, and glycoproteins of the matrix and can activate several other MMPs including collagenase (reviewed in Woessner, 1996).

Matrilysin is frequently expressed in various types of cancer including colon, stomach, prostate, and brain cancers. Previous studies have suggested that matrilysin plays important roles in the progression and metastasis of colon cancer. Recently it has been described by Newell *et al.,* 2002 that there is an increase of matrilysin expression at different stages of UC-associated neoplasia. This work however does not determine whether such increased expression is a result of UC or rather due to the presence of neoplasia.

Membrane associated protein 17 (MAP-17) or otherwise known as DD96 was originally identified as up-regulated in carcinomas with a potential role in modulating cell replication and tumor growth. Newer findings showed that it is also plays a role in surface expression of scavenger receptors class B type I in liver by regulating PDZK1 resulting in a new name SPAP for small PDZK1-associated protein (Identification of small PDZK1-associated protein, DD96/MAP-17, as regulator of PDZK1 and plasma high density lipoprotein levels; Silver et al. J Biol Chem 2003, 278 (31), p28528-32).

Reg IV is a secreted protein and member of the Reg multigene family which have been functionally implicated in regeneration, proliferation and differentiation of the pancreas, the liver and the gastrointestinal mucosa. Reg IV in particular is up-regulated in malignancies of the gastrointestinal tract and is associated with intestinal and neuroendocrine differentiation of stomach and gastric carcinomas (colorectal carcinoma, colon adenocarcinomas); (RegIV activates the epidermal growth factor receptor/Akt/Ap-1 signaling pathway in colon adenocarcinomas; Bishnupuri et al., Gastroenterology 2006 130 (1), p137-49).

Pantetheinase (EC 3.5.1.) is an ubiquitous enzyme which *in vitro* has been shown to recycle pantothenic acid (vitamin B5) and to produce cysteamine, a potent anti-oxidant. The enzyme is encoded by the Vanin-1 gene and is widely expressed in mouse tissues. Vanin-1 is a GPI-anchored pantetheinase, and consequently an ectoenzyme. It has been suggested that Vanin/pantetheinase might be involved in the regulation of some immune functions maybe in the context of the response to oxidative stress (Pitari *et al.,* 2000).

Methods to analyse the quality and quantity of the transcribed mRNA are described in the several laboratory handbooks (for example, in Sambrook and Russell, 2001) and are well known for a person skilled in the art. These methods comprise ribonuclease protection, primer extension, northern blotting, dot blot hybridization, and conventional or real time PCR.

Traditionally, the amount of a particular mRNA produced, and thus the activation status of a gene has been measured by northern blotting. Total RNA isolated from a sample is separated by agarose gel electrophoresis, and probed with a complementary DNA probe specific for the gene of interest. In conventional polymerase chain reaction (PCR), the total RNA isolated from the cell or tissue to be analyzed is reverse transcribed into first strand cDNA (RT-PCR), which is then used as a template to amplify a double stranded amplicon with target specific oligonucleotide primers. In both techniques detection is based on detectable labels, such as fluorescent dyes or radioactive isotopes. Also the recently developed techniques known as DNA chips or microarrays are based on hybridization the target DNA to complementary target specific primers, washing out the unbound DNA and quantifying the bound target DNA. Probes and primers used in the hybridization reactions may be designed based on the nucleotide sequence of the marker gene or amino acid sequence of the translated protein, corresponding the marker gene. A convenient quantitative hybridization method for determining variations in the amounts of expressed RNA is described in the International patent application WO 2002/055734.

Preferably, the "marker gene expression" may be quantified with real time PCR, also called quantitative real time PCR. The method follows the general pattern of polymerase chain reaction, but the amplified region of the target DNA is quantified after each round of amplification by using fluorescent dyes, such as SYBR Green that intercalate with double-stranded DNA or modified DNA oligonucleotide probes that fluoresce when hybridized with a complementary DNA. Frequently, real time PCR is combined with reverse transcription to quantify low abundance mRNA. The data can be analysed by computer software, such as Applied Biosystems 7500 or 7500 Fast Real Time PCR Systems, to calculate relative gene expression between several samples, or mRNA copy number based on a standard curve. Relative quantification (RQ) is commonly used to compare expression levels of wild-type with mutated alleles or the expression levels of a gene in different tissues. RQ determines the change in expression of a target gene in a test sample relative to the same sequence in a basal or calibrator sample (a sample used as the basis for comparative results). The calibrator sample can be an untreated control or a sample at time zero in a time-course study. By using an endogeneous or intrinsic control, it is possible to normalize quantification of a cDNA target for differences in the amount of cDNA added to each reaction. Typically, housekeeping genes such as β-actin, glyseraldehyde-3-phosphate dehydrogenase (GAPDH) and ribosomal RNA (rRNA) are used as endogeneous controls, because their expression levels are relative stable. Replicate reactions per sample and an endogeneous control are needed to ensure statistical significance.

The protein specific antibodies are preferably purified from antiserum obtained by immunizing antigen protein in an animal. More preferably, the protein specific antibodies are polyclonal antibodies purified from the serum obtained by immunizing antigen protein in a rabbit.

To synthesize an antibody specifically recognizing a protein, the protein is first acquired. The protein can be synthesized using known amino acid sequences or produced in recombinant protein types by genetic engineering methods. For example, recombinant protein can be prepared by a method comprising preparing an expression vector of expressing the protein in the form of a recombinant protein using the base sequence of the gene set forth in the NIH program GenBank database; obtaining a transformant by transforming the expression vector into E. *coli* to produce recombinant protein; and cultivating the transformant to isolate/purify the human recombinant protein.

Diagnosing IBD (e.g. UC and CD) by an antigen-antibody binding reaction using the specific antibodies for the proteins enclosed in this invention can be made by determining the expression of these proteins in a sample. The level of expression can be detected by technique known in the art, including enzyme-linked immunosorbent assay (ELISA), radioimmunoassay(RIA), sandwich assay, and Western blotting or immunoblotting analysis on a polyacrylamide gel as well as immunohistochemical assays.

As the sample (specimen), tissues, bodily fluids, most preferably blood and faeces, are preferably used.

One embodiment of the invention is a method for use in the differentiation between inflammatory bowel disease (IBD) and irritable bowel syndrome (IBS) in a patient, wherein the expression levels of at least two markers are determined in a sample taken from said patient, said markers chosen from the group comprising the following genes and their corresponding proteins (Table 1):

**Table 1.**

| Gene: | SEQ.ID.NO. | PROTEIN ACC. NO. |
|---|---|---|
| SLC6A14 | NM_007231 | NP_009162 |
| SLC26A2 | NM_000112 | NP_000103 |
| GRO-1 | N_001511 | NP_001502 |
| MMP-7 | BC003635 | NP_002414 |
| MAP-17 | NM_005764 | NP_005755 |
| RegIV | BC017089 | NP_114433 |
| Vanin-1 | NM_004666 | NP_004657 |

Another embodiment of the invention is a method wherein, in case inflammatory bowel disease is indicated, a differentiation between ulcerative colitis and Crohn's disease is performed by determining the expression levels of at least two markers in a sample taken from said patient, said markers chosen from the group presented in Table 1 above.

Further, according to an embodiment of the invention the disease activity is monitored, and a distinction is made between an active and passive phase of ulcerative colitis or Crohn's disease, based on the expression pattern of said at least one marker; a gene or corresponding protein of fragments thereof. Preferably SLC6A14 and GRO-1 are used, or alternatively or in combination therewith Reg IV and Vanin-1.

In the above methods, said sample is preferably one of a stool sample, blood, plasma, serum and biopsy samples, most preferably a stool sample or a blood sample.

Preferably the determination is first performed in a stool sample, in order to discriminate between IBS and IBD. If IBD is indicated, a further analysis is made on a biopsy sample, in order to discriminate between UC and CD. The analysis of both stool and biopsy samples can be repeated in order to monitor the progression of the disease or the recovery of the patient.

According to an embodiment of the invention, the expression levels are determined using at least two antibodies capable of identifying said at least two proteins or a fragment thereof, contacting at least a portion of said sample with said at least two antibodies; and monitoring the extent of reaction between the contacted sample and the antibodies.

According to an embodiment of the invention, the contacting and monitoring steps are carried out by extracting the proteins from the sample and conducting an assay to determine the quantity of at least two of the listed proteins therein.

Preferably the antigen-antibody binding reaction is detected by any one technique selected from the group consisting of enzyme-linked immunosorbent assay (ELISA), radioimmunoassay (RIA), sandwich assay, Western blotting, immunoblotting analysis or a immunohistochemistry method.

Alternatively, the contacting and monitoring steps are carried out by immunohistochemically reacting the patient sample and the antibodies and then detecting the reactions in the sample.

According to the invention, the antibodies are polyclonal or monoclonal.

Another embodiment of the invention is a kit suitable for use in differentiating between inflammatory bowel disease and irritable bowel syndrome in a patient, said kit comprising at least two antibodies capable of identifying at least two proteins or protein fragments chosen from the group comprising the proteins presented in Table 1 above, and means for detecting the extent of the reaction of the antibodies with a sample.

Another embodiment of the invention is a kit suitable for differentiating between ulcerative colitis and Crohn's disease, preferably comprising at least two antibodies capable of identifying at least two proteins or protein fragments chosen from the group comprising the proteins presented in Table 1 above, detecting the extent of the reaction of the antibodies with the proteins directly in bodily fluids wherein the antibodies are coupled to a solid matrix, and further containing instruction for use.

In a kit according to an embodiment of the invention, the expression pattern of said at least two proteins is compared to a standard pattern in order to determine whether a patient suffering from ulcerative colitis or Crohn's disease suffers from said disease in its active or passive form.

Further, the kit includes the protein, protein fragment, or peptide provided as a control in a known quantity.

According to another embodiment of the invention, the kit is suitable for use in a competitive immunoassay, and further comprises a solid phase, and the protein, protein fragment or peptide fragment is bound to the solid phase. Said solid phase preferably comprises one or more of nitrocellulose, cellulose, glass, plastic, microtitre plates and wells, such as microtitre wells.

According to a preferred embodiment, the ELISA technique using SLC6A14, SLC26A2, GRO-1, MMP-7, MAP-17, Reg IV and Vanin-1 protein specific antibodies is carried out through the following steps:
1) placing a specific antibody into a reactor coated with a sample and a control group to induce an antigen-antibody reaction;
2) detecting an antigen-antibody reaction product using a secondary antibody-label conjugate and a color-developing substrate solution of a labeling substance; and
3) comparing the detection result of the sample with that of the control group.

A large amount of the sample can be analyzed using known technique such as ELISA, biological microchip or automated microarray system. The biological micro chip can detect an antigen for the specific antibody protein by fixing the specific antibody protein on a biological microchip, causing a reaction between the same and a sample collected from an individual.

Also, the present invention provides a kit for diagnosing UC and CD comprising the combination of at least two of these genes using antibodies specifically reacting with the chosen at least two genes.

The diagnostic kit according to the present invention comprises a solid phase or carrier, such as a wicking membrane. Example of materials suitable for such a wicking membrane include, but are not limited to nylon, polyester, cellulose, polysulfone, polyvinylidene difluoride, cellulose acetate, polyurethane, glass fiber, nitrocellulose, or the like. The solid phase can also be in the form of a microcarrier, particles, membranes, strips, paper, film, pearls or plates, such as microtiter plates.

The diagnostic kit can diagnose UC and CD by quantitatively and qualitatively analyzing an antigen of the antibody protein by the antigen- antibody binding reaction. The antigen-antibody binding reaction can be detected by technique generally known in the art, including ELISA, RIA, sandwich assay, Western blotting on a polyacrylamide gel, and immunoblotting analysis. For example, the diagnostic kit can be provided to be used for ELISA using a 96-well microtiter plate coated with recombinant monoclonal antibody protein. Another example would be the use of diagnostic dipsticks for faeces or blood samples.

The controls contained in the diagnostic kit according to the present invention include positive controls and negative controls.

The antibodies used in the diagnosis of the different samples can be polyclonal or monoclonal, and can be made by methods now well-known in the art. For example, polyclonal antibodies made to the extract of Example 2 or 3 can be produced by immunizing an animal such as a rabbit and then purifying the antibodies according to conventional practices, while monoclonal antibodies to said extracts can be made, for example, according to the method of Kohler and Milstein (Nature (1975) 256:495) by immunizing an animal such as a mouse, extracting splenocytes from the spleen thereof, fusing them with mouse myeloma cells to make hybridomas, and screening and subcloning the hybridomas, according to well know practices.

In a preferred embodiment, the immunoassay used in the method of the present invention is of the ELISA sandwich type. In a more preferred embodiment, the ELISA sandwich immunoassay involves the pretreatment of the sample with a specific buffer (clearing buffer) containing a pancreatic cholesterol esterase for decreasing interference of sample circulating lipids in protein quantification. In another preferred embodiment the immunoassay is a Western blot assay (specially preferred with an internal control).

As the secondary antibody labeling substance, known labeling substances inducing color development are preferably used, and examples the labeling substance useful in the present invention include horseradish peroxidase (HRP), alkaline phosphatase, colloidal gold, fluorescein such as poly L-lysine-fluorescein isothiocyanate(FITC) or rhodamine-B-isothiocyanate (RITC), and a dye. In the present invention, goat anti-rabbit IgG-HRP conjugate (IgG-HRP conjugate), for example, is used.

Preferably, the chromogen used varies according to the labeling substance involving the color development, and usable examples thereof include 3,3', 5, 5'-tetramethyl bezidine (TMB), 2,2'-azino-bis (3-ethylbenzothiazoline-6-sulfonic acid) (ABTS), and o-phenylenediamine (OPD).

More preferably, the chromogen is provided in a state in which it is dissolved in a buffer solution (0.1 M NaAc, pH 5.5). The chromogen such as TMB is decomposed by HRP used as the labeling substance of the secondary antibody conjugate to produce a color-developing precipitate. The level of precipitation of the color-developing precipitate is observed by the naked eye, thereby determining the presence of a protein antigen.

The washing solution preferably include a phosphate buffer solution, NaCl, and Tween 20, more preferably a buffer solution containing 0.02 M phosphate buffer solution, 0.13 M NaCl, and 0.05% Tween 20. Following antigen-antibody binding reaction, an appropriate amount of the washing solution is supplied into the reactor having undergone the reaction between the secondary antibody and the antigen-antibody conjugate. Washing with the washing solution is repeatedly performed 3 to 6 times. 0.1 % BSA containing phosphate buffer is preferably used as the blocking solution and a 2 N sulfuric acid solution is preferably used as the enzymatic reaction stopping solution.

The method of diagnosing UC and CD in an early stage by detecting SLC6A14, SLC26A2, GRO-1, MMP-7, MAP-17, Reg IV and Vanin-1 antigens in a specimen sample using the diagnostic kit will now be described. Antibodies and specimen samples in the positive and negative control groups are reacted, respectively, and washed with the washing solution. Then, the secondary antibody conjugate labeled with a labeling substance producing a color by the reaction with the substrate is added to the resultant product and washed again with the washing solution. Then, the substrate containing solution is added to the resultant production to induce color development. Then, the absorbance at 450 nm is measured. Here, the average absorbance of the standard antigen solution A should be greater than or equal to 0.000 and less than or equal to 0.200. The average absorbance of the standard antigen solution F should be greater than or equal to 1.200 and less than or equal to 3.000. The mean value between the absorbance values of the standard antigen solutions A and F is set as a cut-off value to then be used to determine samples as positive or negative samples. When the absorbance of a sample is greater than that of the standard antigen solution F, the sample is diluted and the absorbance thereof is then measured again. The sample having absorbance above the cut-off value is identified as being positive, and the sample having absorbance below the cut-off value is identified as being negative.

It was confirmed that the UC/CD diagnostic kit containing the human specific antibodies for SLC6A14, SLC26A2, GRO-1, MMP-7, MAP-17, Reg IV and Vanin-1 according to the present invention, which is a new immunological diagnostic tool using patient's blood or faeces (biopsy), had higher accuracy and reproducibility. Conventionally, an combination between endoscopy, histology, disease history and P-ANCA antibodies is used for diagnosis of IBD which is, however, not very high in accuracy (Bossuyt, X., Clin Chem 52 2006). However, the test for early diagnosis of UC and CD according to the present invention showed a diagnosing accuracy of about 95 to 97%, which is statistically significantly higher than the conventional tests. Therefore, the diagnosing method and kit according to the present invention can be very advantageously used for early diagnosis of UC and CD and diagnosis of IBS because they have high accuracy and reproducibility.

### Examples

The present invention will now be illustrated in detail by the following examples, without in anyway being limited in scope to the specific embodiments described herein.

### Example 1. Tissue collection and storage

Colon tissue biopsies, faeces and blood samples were taken from patients suffering from UC, CD or IBS and from healthy volunteers.

### 1.1 For RNA work:

Faeces samples were taken from patients suffering from UC, CD or IBS (5 to 10 patients each). The faeces samples were mixed in a 1:1 ratio with RNAlater® solution (Ambion Inc./Applied Biosystems) and kept at RT until use.

### 1.2 For protein work:

Faeces samples were taken from patients suffering from UC, CD or IBS. The faces samples were directly frozen at -20°C until use. Alternatively, the samples are mixed with appropriate buffer in a 1:1 ratio.

### Example 2. RNA extraction from faeces samples for Real-time PCR analysis

### 2.1 RNA isolation:

Total RNA from all patient faeces samples was isolated by first homogenizing the biopsies using a Pellet Pestle Motor Homogenizer according to the manufacturer's protocol (Kimble / Kontes, Kimble Glass Inc.). From the homogenate, total RNA was isolated using Qiagen RNeasy® Kit as according to manufactures guidelines (Qiagen Nordic AB).

### 2.2 cDNA synthesis:

Two micrograms of each RNA sample was used for a first strand cDNA synthesis using 10pM of the Oligo-dT-primer VN-T20 (5'- TTT TTT TTT TTT TTT TTT TTN V-3'). The buffer, deoxynucleotide triphosphates (dATP, dCTP, dGTP and dTTP) and the enzyme reverse transcriptase (Superscript II) were supplied by Invitrogen and the reactions were performed according to the manufactures guidelines. The reaction mixture for first strand synthesis excluding the enzyme was preincubated for 10 min at 65°C in a PCR machine (PCR sprint from Hybaid), chilled on ice, and then the enzyme Superscript II was added and incubated for 1,5 hour at 42°C in a PCR machine.

### 2.3 Realtime PCR analysis of cDNA derived from faeces samples:

Real time PCR analysis was performed on an applied biosystems 7500 PCR machine using 1:10 diluted 1st strand cDNA material derived from each faeces sample and the SYBR Green Mastermix from Applied Biosystems according to the manufacturer's recommendations. Primers were designed to amplify the seven marker genes and internal control, resulting in fragments ranging 106-160bp in size. (Small fragments are necessary as fragments over 400bp tend not to give reliable signals). Using software provided for use with the real-time PCR analysis (SDS 4.0; relative quantification assay), so called "delta Ct" values were obtained for all makers for each faeces sample analyzed.

For this type of analysis special real time PCR primers were used giving the indicated fragment size:

**Table 2.**

| | | | | | |
|---|---|---|---|---|---|
| 1. | γ-actin | 150bp | 5. | MMP7 | 140bp |
| 2. | SLC6A14 | 145bp | 6. | MAP17 | 143bp |
| 3. | SLC26A2 | 106bp | 7. | GISP | 160bp |
| 4. | GRO1 | 125bp | 8. | Vanin-1 | 144bp |

The expression profile was then determined by plotting the delta Ct values for each marker against each marker and analyzing the graph (see results).

The primers, forward and reverse, used in the experiments are presented in Table 3.

**Table 3.**

| **Gene** | **forward primer** | **reverse primer** |
|---|---|---|
| SLC6A14 | 5'- GAG CAA AGA GGT GGA TAT TCT GGC - 3' | 5'- CTC CCC AGT CAG GGT ATG GAA TTG - 3' |
| SLC26A2 | 5'- CAC CTA AAG CTA TTA TGC AGG AGG - 3' | 5'- CTC CTC AAT TCA TGA CCT GTG GGC - 3' |
| GRO-1 | 5'- GCC AAT GAG ATC ATT GTG AAG GCA- 3' | 5'- CAA CAT GAG AAA TGT TGA CCA CAC - 3' |
| MMP-7 | 5'- CAC TGT TCT TCC ACT CCA TTT AGC - 3' | 5'- GAC ATC TAC CCA CTG CAA GTA TAG - 3' |
| MAP17 | 5'- GTT CCT GGT CCT CGT TGC AAT CGC - 3' | 5'- CCA TCG AAG AGT ACC TTC CAT CTG - 3' |
| Reg IV | 5'- GGT GAT ATC ATC ATG AGA CCC AGC - 3' | 5'- CTT TAA ACT CAG GAT AGA TGC CAG - 3' |
| Vanin-1 | 5'- CCA ACT GAC TGA TAG ACT CTG AGC - 3' | 5'- GGC ATA GAT CAC TAC TGC AAG TGC - 3' |
| γ-Actin | 5'- GTG CAG GGT ATT AAC GTG TCA GGG - 3' | 5'- CCA ACT CAA AGC AAG TAA CAG CCC ACG G - 3' |

The RNA data show that GRO-1 and Reg IV are a good combination for discrimination of IBD and IBS on RNA level in faeces (See e.g. Fig. 5). Further, RNA data from blood samples shows that Vanin-1 has a good discriminating potential has. The markers SLC26A2, MAP-17 and Vanin-1 are well detectable in blood on the RNA level. The markers GRO-1, MMP-7 and RegIV. SLC14A6 were detectable, but on a lower level, when expressed in blood on RNA level.

From the fact that MMP-7, RegIV and GRO-1 are secretory proteins, the inventors conclude that the expression on protein level would be easier to detect, and this could be used to confirm the performance of the blood RNA data.

Regarding RNA in blood, it should be noted that the discrimination of IBD and IBS was reliable already using only one marker, Vanin-1. The AUC was 0.867.

### Example 3. Protein extraction

### 3.1 Protein extraction from biopsy samples

The obtained fixed biopsy samples have to be centrifuged, the fixative removed and washed with PBS. Then extraction buffer (100mM potassium phosphate pH 7,8; 1 mM DTT; 0,5mM PMSF) was added and the sample homogenized using a Pellet Pestel Homogenizer according to the manufacturer's protocol. Then 0,1-0,2% of Triton-X-100 was added and the mixture incubated for 5 min at 4°C to lyse the cells. After centrifugation of 5min at 4°C the supernatant (=extract) was transferred into a new tube and the extract stored after shock freezing at -80°C until usage.

### 3.2 Protein extraction from faeces samples

Extraction buffer (100mM potassium phosphate pH 7,8; 1 mM DTT; 0,5mM PMSF) and glass beads were added to the faeces samples and thoroughly vortexed. Then 0,1-0,2% of Triton-X-100 was added and incubated for 5 min at 4°C to lyse the cells. After centrifugation during 5 min at 4°C , the supernatant (=extract) was transferred into a new tube and the extract stored at -80°C until use

### 3.3 Protein extraction from blood

To the frozen serum the same amount of 2x extraction buffer (200mM potassium phosphate pH 7,8; 2mM DTT; 1 mM PMSF) plus 0,2-0,4% of Triton-X-100 was added and the mixture incubated for 5 min at 4°C to lyse the cells. After centrifugation during 5min at 4°C , the supernatant (=extract) was transferred into a new tube and the extract shock frozen and stored at -80°C until usage.

Instead of extracting the proteins from the blood or the serum, the serum can be used directly to coat the ELISA plates.

### Example 4. Western Blot Analysis of the specimen samples with polyclonal antibodies

For each patient and specimen sample, the protein extract (standardized to 10 µg of total protein/lane) were separated eight times by 12 or 15% denaturing SDS-PAGE and transferred to a polyvinylidene difluoride membrane (Hybond-P; 0.8 mA/cm.sup.2 for 60 min; Amersham Pharmacia Biotech) by semidry blotting using an electroblotter (Bio-Rad). The membrane was then dissected into eight stripes for reaction with specific antibodies for the proteins of PROT. ID. 1 - 7 and beta-tubulin. Antibodies were diluted in blocking buffer. Membranes were subsequently washed, incubated with ECL-Plus Detection Reagent, and exposed to Hyperfilm ECL (both from Amersham Pharmacia Biotech). Primary antibodies directed against Reg IV and MMP-7 were obtained from Abcam, GRO-1 from RDS and SLC6A14, SLC26A2, MAP-17 and Vanin-1 were synthesized by commercial suppliers. Protein contents were normalized by parallel hybridization with an antibody against alpha-tubulin. All Western blots were exposed to film for varying lengths of time, and only films generating subsaturating levels of intensity were selected for densitometrical and statistical evaluation. Linearity was assured in independent experiments by using different amounts of material and multiple film exposures (data not shown).

As a positive control the recombinant proteins of PROT. ID. 1-7 (obtained from the same source as the antibodies) were also analysed in the same way.

### Example 5: ELISA analysis using polyclonal antibodies raised against the marker proteins

First, wells were coated with the sample. Second, ELISA plate wells were coated with the specific polyclonal antibodies to the seven proteins, PROT ID NO 1 - 7. The third step was the detection of the presence of specific antigen-antibody binding in the samples.

### 5.1 Coating with sample on the wells

Protein extracts obtained from colon biopsies, faeces and blood samples were used as specimen samples. To obtain a standard curve for measurement of cut-off values, standard antigen solutions 1 to 7 were prepared with dilutions A, B, C, D, E, F and G at various concentrations of 0ng/ml, 20 ng/ml, 40 ng/ml, 80 ng/ml, 160 ng/ml, 320 ng/ml and 640 ng/ml, respectively.

100 µl of the respective specimen protein samples were distributed to respected 96-well ELISA flat-bottomed plate and reacted at 37°C for 4 hours, followed by washing 4 times with a washing buffer solution (PBS including 0.05% Tween 20, Ph 7.4). Here, the standard antigen solutions prepared above were used as positive control groups and normal rabbits sera were used as negative control groups.

### 5.2 Addition of the polyclonal antibodies

Specific polyclonal antibodies to the proteins of PROT. ID 1-7 were placed in each well coated with the specimen samples, covered with a lid and allowed to stand at 4 C for 16 to 18 hours. The polyclonal antibodies were diluted in 0.5 M carbonate buffer (pH 9.6) in a concentration of 5 µg/ml and 100 µl of the diluted solution was added to each well. As a control group, the normal rabbit serum that was not infected with the proteins was 500-fold diluted in a carbonate buffer solution and distributed to each well (100 pl/well).

Then, the wells of the plate were washed 4 times with a washing buffer solution. In order to block non-specific protein binding sites, a blocking solution (PBS buffer solution (pH 7.4) containing a 2% BSA) was distributed to each well (300pl/well) and allowed to stand at 37°C at 2 hours.

### 5.3 Detection of Antigen-Antibody Complex

100 µl of a 10,000-fold dilution of horseradish peroxidase conjugated goat anti-rabbit IgG secondary antibody was added to each well, and the plate was allowed to stand at 37°C for 1 hour, followed by washing 4 times with a washing buffer solution. Subsequently, 1 mg of 3.3', 5.5'-tetramethylbenzidine (TMB) (Sigma Co., USA) as a substrate was dissolved in 10 ml of a citrate buffer solution (pH 5.0) and 2µl of 35% hydrogen peroxide was added thereto, thereby preparing a substrate solution.100 ul of the prepared substrate solution was distributed to each well and reacted at room temperature for 15 minutes without exposure to light. Thereafter, 50 µl of a 2 N H2SO4 solution was added to stop the reaction and the absorbance at 450 nm was measured.

For each specimen sample tested with one of the proteins of PROT. ID 1-7, the absorbance by the antigen was inferred as the remainder obtained by subtracting the absorbance of wells coated with only the protein as the positive control group and PBS as the negative control group from the absorbance of the sample.

In the same manner, after the absorbance values of the standard solutions were calculated the mean value between the absorbance values of the standard antigen solutions A and F for each protein was set as a cut-off value. The sample having absorbance above the cut-off value was identified as being positive, and the sample having absorbance below the cut-off value was identified as being negative.
Based on the cut-off value, the absorbance values of the respective samples were compared to determine whether they are positive or negative.

Although the invention has been described with regard to its preferred embodiments, which constitute the best mode presently known to the inventors, it should be understood that various changes and modifications as would be obvious to one having the ordinary skill in this art may be made without departing from the scope of the invention as set forth in the claims appended hereto.

### References

KS Bishnupuri LQ, N Murmu, C W Houchen, S Anant, B K Dieckgraefe. Reg IV activates the epidermal growth factor receptor/Akt/AP-1 signaling pathway in colon adenocarcinomas. Gastroenterology 2006;130:137-49.
Bossuyt X. Serologic Markers in Inflammatory Bowel Disease. Clin Chem. 2006; 52:171-181
Heller et al., Discovery and analysis of inflammatory disease-related genes using cDNA microarrays, Proc Natl Acad Sci U S A. 1997 Mar 18;94(6):2150-5.
Hirata I, Murano M, Nitta M, Sasaki S, Toshina K, Maemura K, Katsu K (2001). Estimation of mucosal inflammatory mediators in rat DSS-induced colitis. Possible role of PGE(2) in protection against mucosal damage. Digestion. 63 Suppl 1:73-80
Isaacs KL, Sartor RB, Haskill S (1992). Cytokine messenger RNA profiles in inflammatory bowel disease mucosa detected by polymerase chain reaction amplification. Gastroenterology. 103:1587-95
Keiichi Mitsuyama OT, Nobuo Tomiyasu, Koseku Takaki, Asuka Suzuki, Junya Masuds Hiroshi Yamasaki, Atsushi Toyonaga, Michio Sata. Increased Circulating Concentrations of Growth-Related Oncogene (GRO)- α in Patients with Inflammatory Bowel Disease. Digestive Diseases and Sciences 2006;51:173-77
Kohler and Milstein (Nature (1975) 256:495)
Lawrance IC, Fiocchi C, Chakravarti S (2001). Ulcerative colitis and Crohn's disease: distinctive gene expression profiles and novel susceptibility candidate genes. Hum Mol Genet. 10:445-56.
Newell KJ et al., Matrilysin (matrix metalloproteinase-7) expression in ulcerative colitis-related tumorigenesis, Mol Carcinog. 2002 Jun;34(2):59-63.
Pitari G et al.., Pantetheinase activity of membrane-bound Vanin-1: lack of free cysteamine in tissues of Vanin-1 deficient mice, FEBS Lett. 2000 Oct 20;483(2-3):149-54
Silver DL, Wang N, Vogel S. Identification of Small PDZK1-associated Protein, DD96/MAP17, as a Regulator of PDZK1 and Plasma High Density Lipoprotein Levels. The Journal of Biological Chemistry 2003;278:28528-532.
Spandau et al., High expression of chemokines Gro-alpha (CXCL-1), IL-8 (CXCL-8), and MCP-1 (CCL-2) in inflamed human corneas in vivo, Ophthalmol. 2003 Jun;121(6):825-31.
Woessner JF Jr, Regulation of matrilysin in the rat uterus, Biochem Cell Biol. 1996;74(6):777-84.

## Claims

1. A kit for use in a method for differentiation between IBD and IBS comprising
at least two antibodies directed to at least two proteins or protein fragments chosen from the group comprising the following proteins:
| Gene: | PROTEIN ACC. NO. |
|---|---|
| SLC6A14 | NP_009162 |
| SLC26A2 | NP_000103 |
| GRO-1 | NP_001502 |
| MMP-7 | NP_002414 |
| MAP-17 | NP_005755 |
| RegIV | NP_114433 |
| Vanin-1 | NP_004657 |
and
means for detecting the extent of the reaction of the antibodies with a sample.

2. The according to claim 1, further comprising instructions for using said kit for differentiating between IBD and IBS.

3. The kit according to claim 1 or 2, wherein said at least two antibodies are selected from the group consisting of antibodies directed to the following proteins: SLC6A14, SLC26A2, GRO-1, MMP-7, MAP-17, RegIV and Vanin-1.

4. The kit according to claim 3, wherein one of said at least two antibodies is the antibody directed to the SLC26A2 protein.

5. The kit according to claim 4, wherein one of said at least two antibodies is the antibody directed to the GRO-1 protein.

6. The kit according to claim 4, wherein one of said at least two antibodies is the antibody directed to the MAP-17 protein.

7. The kit according to claim 4, wherein one of said at least two antibodies is the antibody directed to the RegIV protein.

8. The kit according to claim 4, wherein one of said at least two antibodies is the antibody directed to the SLC6A14 protein.

9. The kit according to claim 4, wherein said at least two antibodies are the antibodies directed to the GRO-1 and SLC26A2 proteins.

10. The kit according to claim 4, wherein said at least two antibodies are the antibodies directed to the GRO-1 and SLC6A14 proteins.

11. The kit according to claim 4, wherein said at least two antibodies are the antibodies directed to the GRO-1 and MAP-17 proteins.

12. The kit according to claim 4, wherein said at least two antibodies are the antibodies directed to the GRO-1 and MMP-7 proteins.

13. The kit according to claim 4, wherein said at least two antibodies are the antibodies directed to the GRO-1 and RegIV proteins.

14. The kit according to claim 4, wherein one of said at least two antibodies is the antibody directed to the Vanin-1 protein.

15. The kit according to any one of the preceding claims, wherein a protein, protein fragment, or peptide is provided to be used as a control in a known quantity.

16. The kit according to any one of the preceding claims, wherein the kit is adapted for use in a competitive immunoassay, and further comprises a solid phase, and the protein, protein fragment or peptide fragment is bound to the solid phase.

17. The kit according to claim 16, wherein the kit is adapted for use in a competitive immunoassay, and further comprises a solid phase, and the protein, protein fragment or peptide fragment being bound to said solid phase; and said solid matrix is chosen among nitrocellulose, cellulose, glass, plastic, microtitre plates and wells.


SEQUENCE LISTING

<110>  Index Diagnostics AB

<120>  METHOD AND KIT FOR USE IN THE DIFFERENTIATION OF IBD AND IBS AND
       FURTHER DISTINCTION BETWEEN DISEASE TYPES OF IBD

<130>  58567EP

<140>  EP 07861152.2
<141>  2007-12-20

<160>  31

<170>  PatentIn version 3.5

<210>  1
<211>  4520
<212>  DNA
<213>  Homo sapiens

<220>
<221>  misc_feature
<222>  (1)..(4520)
<223>  Gene SLC6A14

<300>
<308>  NM_007231.1
<309>  1999-10-01
<313>  (1)..(4520)

<400>  1
taggaacagg ggagagtgca cctgctacca gtcaagctca gccagactgc aagaggaggc      60

gaggcggagc cagccgaggg agtgaaccat ggacaagttg aaatgcccga gtttcttcaa     120

gtgcagggag aaggagaaag tgtcggcttc atcagagaat ttccatgttg gtgaaaatga     180

tgagaatcag gaccgtggta actggtccaa aaaatcggat tatcttctat ctatgattgg     240

atacgcagtg ggattaggaa atgtgtggag atttccatat ctgacctaca gcaatggtgg     300

aggcgccttc ttgatacctt atgcaattat gttagcattg gctggtttac ctttgttctt     360

tctggagtgt tcactgggac aatttgctag cttaggtcca gtttcagttt ggaggattct     420

tccattgttt caaggtgtgg gaattacaat ggtcctgatc tccatttttg tgacaatcta     480

ttacaatgtc ataattgcct atagtcttta ctacatgttt gcttcttttc aaagtgaact     540

accatggaaa aattgttctt cgtggtcaga taaaaactgt agcagatcac caatagtaac     600

tcactgtaat gtgagtacag tgaataaagg aatacaagag atcatccaaa tgaataaaag     660

ctgggtagac atcaacaatt ttacctgcat caacggcagt gaaatttatc agccagggca     720

gcttcccagt gaacaatatt ggaataaagt ggcgctccaa cggtcaagtg gaatgaatga     780

gactggagta attgtttggt atttagcact ttgtcttctt ctggcttggc tcatagttgg     840

agcagcacta tttaaaggaa tcaaatcgtc tggcaaggtg gtatatttta cagctctttt     900

cccctatgtg gtcctactca tcctgttagt acgaggtgca actctggagg gtgcttcaaa     960

aggcatttca tactatattg gagcccagtc aaattttaca aaacttaagg aagctgaggt 1020

atggaaagat gctgccactc agatatttta ctccctttca gtggcttggg gtggcttagt 1080

tgctctatca tcttacaata agttcaaaaa caactgcttc tctgatgcca ttgtggtttg 1140

tttgacaaac tgtctcacta gcgtgtttgc tggatttgct attttttcta tattgggaca 1200

catggcccat atatctggaa aggaagtttc tcaagttgta aaatcaggtt ttgatttggc 1260

attcattgcc tatccagagg ctctagccca actcccaggt ggtccatttt ggtccatatt 1320

attttttttc atgcttttaa ctttgggtct cgattctcag tttgcttcga ttgaaacgat 1380

cacaacaaca attcaagatt tatttcccaa agtgatgaag aaaatgaggg ttcccataac 1440

tttgggctgc tgcttggttt tgtttctcct tggtctcgtc tgtgtgactc aggctggaat 1500

ttactgggtt catctgattg accacttctg tgctggatgg ggcattttaa ttgcagctat 1560

actggagcta gttggaatca tctggattta tggagggaac agattcattg aggatacaga 1620

aatgatgatt ggagcaaaga ggtggatatt ctggctatgg tggagagctt gctggtttgt 1680

aattacgcct atccttttga ttgcaatatt tatctggtca ttggtgcaat ttcatagacc 1740

taattatggc gcaattccat accctgactg gggagttgct ttaggctggt gtatgattgt 1800

tttctgcatt atttggatac caattatggc tatcataaaa ataattcagg ctaaaggaaa 1860

catctttcaa cgccttataa gttgctgcag accagcttct aactggggtc catacctgga 1920

acaacatcgt ggggaaagat ataaagacat ggtagatcct aaaaaagagg ctgaccatga 1980

aatacctact gttagtggca gcagaaaacc ggaatgagat ctcattgaaa aaaatatatg 2040

attgtataat gtgatttttt ttagaatagg gggaacctta tttatttgtg tgttaactga 2100

ataggaaaat gtacatacta tgttcatgat agtgtgattt ttttcacatt taagcaggaa 2160

tgcaatataa aaatgtgaat ctcttaattc tcagccatgt gcttattata tttcttttta 2220

gattgtctat ctgtataaca cacacacaca cacctaagag tctctatttc acaattatat 2280

ttttgtaaat agtatatgca tttttaatac attggaggct ttattttgaa ctaatttctt 2340

agagaatagt tatattttct attacacaag tttaaaaata ttattaactt gtattttctt 2400

aatatacaat ctatcttttc cacaaatatg agtgggaaat aaatcagcac atttgaaaga 2460

aagtgttaaa actgaaggcc tcacttaatt agaaacgtga taaatatatg gacaaatgga 2520

ctatacatac tataagagga ctgtagttta atacttttta cccaaatatg tttaaaaaca 2580

tcgtgcattt gttacagctc atgttttcta tatgaactta gtcattaatg ttctttataa 2640

aaagtgaaat aagatggaaa aattaggatc ctacagccag tacgtgataa atctagaaaa 2700

ttgagttttg agtacctctt ttcccatata caatcttcct tccttaggta atttggaaga 2760

aaactatgac ccatttaatt tctattgtgt ttcaccaaat tcagtgttgt tcattatacc 2820

tctctgaaat ataggtttaa tttcaaatag aatatggact taaatgttaa tgagaaactg 2880

gctttaatca attctagcat tttattactg taatacaggg ctgatagagt gattttgtct 2940

tatatgagtc agttactact tacaggtgat aacttgcata ctattggaag ataaagttgt 3000

caaacttgtc aagaatgaga aaagccaaat tagaaaatcc tatgtcctag tttccttacc 3060

aaggataatt aaatatatca ctaagagctt tatatattga ttatatattg ttgacaactg 3120

gtttaagcat catagcctat gatgataaac actgcctata tatgtaaata gcttttcatc 3180

aattcttaaa tttcttaacc taggcttcag ggagcatatg aaaccaaaat tatatggaac 3240

attttctgtg tgtacatgta catgcatttt tctagggaga gagtccgtag gtttatcaga 3300

atatcaagga aaactgtgac ccaaagaagt ttaagaatca catacagtgc tgctggcttt 3360

ttgtgcttgg caaatgagtg acaatagaag aaataatttt tcttacacat tttaaaacgt 3420

tttctcttcc ttgtgattga agatgaaagg agtaagaaat taaggcattt gtttaattta 3480

tactggtaac ttatttaggg gggaggggac atgaaggtag gtaaataggt aggcctctaa 3540

ttgaaccacc tctctaagtt atgtacgtat atataagctg aaattgtgtt tgacattctg 3600

agggttttct ttttcttttt cctttttttt tttttttggt ggggggctgg gggtcagagt 3660

cttgttctgt tgcctgggct ggagtgcagt ggcatgatct cagctcactg caacctctgc 3720

cttctggatt caagtgattc tcctgcctca gcctcttgag tagctgggac tacaggtgcc 3780

cgccaccaca ccagctaatt tttgtatttt tagtagaggc gaagtttccc catgttggcc 3840

aggctggtct tgaactcccg acctcaagtg atctgtctac ctcggcctcc taaagtgctg 3900

agattacagg tgtgagccac cgtgcccggc ccattctaag ggttttcttt gaagacaggt 3960

caaatgctgt tagtaagttt caggagattg ttaattcctc agttatacca gattttataa 4020

aatatttgag aatagatggc taacaagagg ttagaaatac ttttccttaa ttttaatcca 4080

cagtatgtta catgcattct accactacat tttggtgcta tttaaggtgt gcaattttct 4140

ataggtgact tttgcaattc agggaagatt tgggcatatt aaatgaaaga atatctaatt 4200

gggggaggtg tgaagggaaa gaaattcttt tcaaaagctg accacaaaga gtagttaaaa 4260

gtttttgtca ctatcttcac aagtgtgtaa agcacagatt tcaacagagt gcttggcata 4320

ttgtagggtg ctcaatggtg gtttttatta ttattactca gattccacag tggcaagaaa 4380

catcattcta cataatggaa aacatttaca tcaaatccca cttactttaa tgcgaacttg 4440

gagataattt atggtattgt attgtaaacc attaatgaaa actttttcac agttgagtga 4500

aattaaaatc actatatctc 4520

<210> 2
<211> 642
<212> PRT
<213> homo sapiens

<300>
<308> NP_009162.1
<309> 1999-10-01
<313> (1)..(642)

<400>  2

Met Asp Lys Leu Lys Cys Pro Ser Phe Phe Lys Cys Arg Glu Lys Glu
1               5                   10                  15

Lys Val Ser Ala Ser Ser Glu Asn Phe His Val Gly Glu Asn Asp Glu
            20                  25                  30

Asn Gln Asp Arg Gly Asn Trp Ser Lys Lys Ser Asp Tyr Leu Leu Ser
        35                  40                  45

Met Ile Gly Tyr Ala Val Gly Leu Gly Asn Val Trp Arg Phe Pro Tyr
    50                  55                  60

Leu Thr Tyr Ser Asn Gly Gly Gly Ala Phe Leu Ile Pro Tyr Ala Ile
65                  70                  75                  80

Met Leu Ala Leu Ala Gly Leu Pro Leu Phe Phe Leu Glu Cys Ser Leu
                85                  90                  95

Gly Gln Phe Ala Ser Leu Gly Pro Val Ser Val Trp Arg Ile Leu Pro
            100                 105                 110

Leu Phe Gln Gly Val Gly Ile Thr Met Val Leu Ile Ser Ile Phe Val
        115                 120                 125

Thr Ile Tyr Tyr Asn Val Ile Ile Ala Tyr Ser Leu Tyr Tyr Met Phe
    130                 135                 140

Ala Ser Phe Gln Ser Glu Leu Pro Trp Lys Asn Cys Ser Ser Trp Ser
145                 150                 155                 160

Asp Lys Asn Cys Ser Arg Ser Pro Ile Val Thr His Cys Asn Val Ser
                165                 170                 175

Thr Val Asn Lys Gly Ile Gln Glu Ile Ile Gln Met Asn Lys Ser Trp
            180                 185                 190

Val Asp Ile Asn Asn Phe Thr Cys Ile Asn Gly Ser Glu Ile Tyr Gln
        195                 200                 205

Pro Gly Gln Leu Pro Ser Glu Gln Tyr Trp Asn Lys Val Ala Leu Gln
    210                 215                 220

Arg Ser Ser Gly Met Asn Glu Thr Gly Val Ile Val Trp Tyr Leu Ala
225                 230                 235                 240

Leu Cys Leu Leu Leu Ala Trp Leu Ile Val Gly Ala Ala Leu Phe Lys
                245                 250                 255

Gly Ile Lys Ser Ser Gly Lys Val Val Tyr Phe Thr Ala Leu Phe Pro
            260                 265                 270

Tyr Val Val Leu Leu Ile Leu Leu Val Arg Gly Ala Thr Leu Glu Gly
        275                 280                 285

Ala Ser Lys Gly Ile Ser Tyr Tyr Ile Gly Ala Gln Ser Asn Phe Thr
    290                 295                 300

Lys Leu Lys Glu Ala Glu Val Trp Lys Asp Ala Ala Thr Gln Ile Phe
305                 310                 315                 320

Tyr Ser Leu Ser Val Ala Trp Gly Gly Leu Val Ala Leu Ser Ser Tyr
                325                 330                 335

Asn Lys Phe Lys Asn Asn Cys Phe Ser Asp Ala Ile Val Val Cys Leu
            340                 345                 350

Thr Asn Cys Leu Thr Ser Val Phe Ala Gly Phe Ala Ile Phe Ser Ile
        355                 360                 365

Leu Gly His Met Ala His Ile Ser Gly Lys Glu Val Ser Gln Val Val
    370                 375                 380

Lys Ser Gly Phe Asp Leu Ala Phe Ile Ala Tyr Pro Glu Ala Leu Ala
385                 390                 395                 400

Gln Leu Pro Gly Gly Pro Phe Trp Ser Ile Leu Phe Phe Phe Met Leu
                405                 410                 415

Leu Thr Leu Gly Leu Asp Ser Gln Phe Ala Ser Ile Glu Thr Ile Thr
            420                 425                 430

Thr Thr Ile Gln Asp Leu Phe Pro Lys Val Met Lys Lys Met Arg Val
        435                 440                 445

Pro Ile Thr Leu Gly Cys Cys Leu Val Leu Phe Leu Leu Gly Leu Val
    450                 455                 460

Cys Val Thr Gln Ala Gly Ile Tyr Trp Val His Leu Ile Asp His Phe
465                 470                 475                 480

Cys Ala Gly Trp Gly Ile Leu Ile Ala Ala Ile Leu Glu Leu Val Gly
                485                 490                 495

Ile Ile Trp Ile Tyr Gly Gly Asn Arg Phe Ile Glu Asp Thr Glu Met
            500                 505                 510

Met Ile Gly Ala Lys Arg Trp Ile Phe Trp Leu Trp Trp Arg Ala Cys
        515                 520                 525

Trp Phe Val Ile Thr Pro Ile Leu Leu Ile Ala Ile Phe Ile Trp Ser
    530                 535                 540

Leu Val Gln Phe His Arg Pro Asn Tyr Gly Ala Ile Pro Tyr Pro Asp
545                 550                 555                 560

Trp Gly Val Ala Leu Gly Trp Cys Met Ile Val Phe Cys Ile Ile Trp
                565                 570                 575

Ile Pro Ile Met Ala Ile Ile Lys Ile Ile Gln Ala Lys Gly Asn Ile
            580                 585                 590

Phe Gln Arg Leu Ile Ser Cys Cys Arg Pro Ala Ser Asn Trp Gly Pro
        595                 600                 605

Tyr Leu Glu Gln His Arg Gly Glu Arg Tyr Lys Asp Met Val Asp Pro
    610                 615                 620

Lys Lys Glu Ala Asp His Glu Ile Pro Thr Val Ser Gly Ser Arg Lys
625                 630                 635                 640

Pro Glu

<210>  3
<211>  8082
<212>  DNA
<213>  homo sapiens

<220>
<221>  gene
<222>  (1)..(8082)
<223>  Gene SLC26A2

<300>
<308>  NM_000112.3
<309>  2006-05-25
<313>  (1)..(8082)

<400>  3
gtgacggcct cggccgcggg cgtttacact ggctctgcct ccggcatctc ttcgccggtg      60

cgtcctcgcc gcgcccgtag gtcccggcag ccgggccccg cctccttcgg agtccgagcg     120

atgggcgggg aaagggacag gcaggtatag ctctgtcggc gccgcggtgt ccacctcagt     180

caggccacgg tggaagacgc gtgccgcggc gcctggttgc ctgcagcggc ccggacccga     240

gaggaagctg aaccatctat ctccagaaat gtcttcagaa agtaaagagc aacataacgt     300

ttcacccaga gactcagctg aaggaaatga cagttatcca tctgggatcc atctggaact     360

tcaaagggaa tcaagtactg acttcaagca atttgagacc aatgatcaat gcagacctta 420

tcataggatc cttattgagc gtcaagagaa atcagataca aacttcaagg agtttgttat 480

taaaaagctg cagaagaatt gccagtgcag tccagccaaa gccaaaaata tgattttagg 540

tttccttcct gttttgcagt ggctcccaaa atacgaccta aagaaaaaca ttttagggga 600

tgtgatgtca ggcttgattg tgggcatatt attggtgccc cagtccattg cttattccct 660

gctggctggc caagaacctg tctatggtct gtacacatct ttttttgcca gcatcattta 720

ttttctcttg ggtacctccc gtcacatctc tgtgggcatt tttggagtac tgtgccttat 780

gattggtgag acagttgacc gagaactaca gaaagctggc tatgacaatg cccatagtgc 840

tccttcctta ggaatggttt caaatgggag cacattatta aatcatacat cagacaggat 900

atgtgacaaa agttgctatg caattatggt tggcagcact gtaaccttta tagctggagt 960

ttatcaggta gcgatgggct tctttcaagt gggttttgtt tctgtctacc tctcagatgc 1020

cttgctgagt ggatttgtca ctggtgcctc cttcactatt cttacatctc aggccaagta 1080

tcttcttggg ctcaaccttc ctcggactaa tggtgtgggc tcactcatca ctacctggat 1140

acatgtcttc agaaacatcc ataagaccaa tctctgtgat cttatcacca gccttttgtg 1200

ccttttggtt cttttgccaa ccaaagaact caatgaacac ttcaaatcca agcttaaggc 1260

accgattcct attgaacttg ttgttgttgt agcagccaca ttagcctctc attttggaaa 1320

actacatgaa aattataatt ctagtattgc tggacatatt cccactgggt ttatgccacc 1380

caaagtacca gaatggaacc taattcctag tgtggctgta gatgcaatag ctatttccat 1440

cattggtttt gctatcactg tatcactttc tgagatgttt gccaagaaac atggttacac 1500

agtcaaagca aaccaggaaa tgtatgccat tggcttttgt aatatcatcc cttccttctt 1560

ccactgtttt actactagtg cagctcttgc aaagacattg gttaaagaat caacaggctg 1620

ccatactcag ctttctggtg tggtaacagc cctggttctt ttgttggtcc tcctagtaat 1680

agctcctttg ttctattccc ttcaaaaaag tgtccttggt gtgatcacaa ttgtaaatct 1740

acggggagcc cttcgtaaat ttagggatct tcccaaaatg tggagtatta gtagaatgga 1800

tacagttatc tggtttgtta ctatgctgtc ctctgcactg ctaagtactg aaataggcct 1860

acttgttggg gtttgttttt ctatattttg tgtcatcctc cgcactcaga agccaaagag 1920

ttcactgctt ggcttggtgg aagagtctga ggtctttgaa tctgtgtctg cttacaagaa 1980

ccttcagatt aagccaggca tcaagatttt ccgctttgta gcccctctct actacataaa 2040

caaagaatgc tttaaatctg ctttatacaa acaaactgtc aacccaatct taataaaggt 2100

ggcttggaag aaggcagcaa agagaaagat caaagaaaaa gtagtgactc ttggtggaat 2160

ccaggatgaa atgtcagtgc aactttccca tgatcccttg gagctgcata ctatagtgat 2220

tgactgcagt gcaattcaat ttttagatac agcagggatc cacacactga aagaagttcg 2280

cagagattat gaagccattg gaatccaggt tctgctggct cagtgcaatc ccactgtgag 2340

ggattcccta accaacggag aatattgcaa aaaggaagaa gaaaaccttc tcttctatag 2400

tgtgtatgaa gcgatggctt ttgcagaagt atctaaaaat cagaaaggag tatgtgttcc 2460

caatggtctg agtcttagta gtgattaatt gagaaggtag atagaagaat gtctagccaa 2520

taggttaaaa tttcaagtgt ccaacatttc ccagttccac agtgggaaat tttgcacact 2580

tgaaatttta accaagtggc tagatattat tcctcctttg aagctaatgg catttgtata 2640

tacacactgc agcagagctt gtagctggac agagtcaaaa agaagaaaat acggtttcag 2700

gctttcttgc agatatgaag tattcttgga atgcaataag tatgtattga actgtactgt 2760

aaagtagctc caaaacttaa ttactctcct gttttagggg ttatacattt ggactgtgca 2820

ttctccaaga gatgaagcgg tgaagttggg atttacattg gaagtgctgt agacttcttt 2880

atgtggctca gtggagagag ggaaagaatg ttgcacctgc tctagtacca taggtcaaga 2940

ggcttctgga tcacaaagtc ataactagac aggtttgttc ttgtagtttt ctatccccag 3000

tctttgctcc ccagatggca gtagttttta gtaggaaagt gccattcctg tccttaaggc 3060

acagtctcat cagaagtcta atacctgggc aggtttataa catcctgaga gccagcctga 3120

cattagacag aataccettt gtaatacatt ggaaattttt actcatgcct ttttgtttag 3180

gataaatagg taagcacaaa gagctcttca aaatcagaaa aaacaatagg agtccttcct 3240

tgtcttttct gtgatctctg tccttgtttc tgagactttc tctaccatta agctctattt 3300

tagctttcag ttattctagt ttgtttccca tggaatctgt cctaaactgg tgtttttgtc 3360

agtgacagtc ttgccagtca gcaatttcta acagcatttt aaatgagttt gatgtacagt 3420

aaatattgat gacaatgaca gcttttaact cttcaagtca cctaaagcta ttatgcagga 3480

ggatttagaa gtcacattca taaaacccaa gtgctatggg tgtattattc atgatagctg 3540

gcccacaggt catgaattga ggaggaattt gctttcaaaa agcaagaatg tccaacactg 3600

aaagtttata gttttatatt tggaccttga aaggtaagaa aaaaccaggt tctccaaagt 3660

taggaatagg gaactaattt atgaaacagc catcttaaaa aaaaaaaaag taaactgcaa 3720

aagtacaaaa tcatttttca atctgttccc agtttctaaa caattttaaa tatttatgag 3780

aagcaaaccc tatgtgtagg gcatctgttg gagtgggatg cttttagaca tatattaagt 3840

atgtacatgt ttaatatgta tatttaaaat gcatatatat tttattatat ctatattatc 3900

ctatatagat atatgtaact tagctttatt gttagctcca taagctgcca gtgttgcttt 3960

tctgttggta gagctctccc atttggtgac atggaaaata cctttccatt atcacaacaa 4020

agcagttgct cagtagaaag tctagatttc tgtcttatag gtgatttctg tcttataggt 4080

gattataatc aagtgtaggc ttcctgaatt ttgacatcct tttagaactt gggtctggaa 4140

ttccagaaat gttaattgct gcttgtattt gttcttgttt gttttttagc cagtatttgc 4200

cctttctatc cagccttatg aataatagca gtaaaatcac agtatcttgg tcagtcttta 4260

tttttttcct tttttctttt ttaagagaca gtcatccagg ccagagtgca gtttgatgat 4320

agcttactga agcttcccac tcctgggctc aagttatcct tccattttgg cctcctgagt 4380

agctagacca taggtatgca tcaccacacc ctgctaattt tttaaatttt tttctagaga 4440

gagggtctca ctgtgttgcc caggctggtc tcaaactcca ggctcaagca atccttcagc 4500

ctcagcctcc cagagtgttg ggattacagg cgtgagccac tgcacttggc caagttattt 4560

atttttaatc tctcttgccc ttctcccaag gcaggcttaa gttgagacta ttataggtgt 4620

ctaataacct gtgacagagt aatgagtaca tgcttaagat gttataatta gccaacacca 4680

acacagcaaa aaatataatt ccagccaaag attctggaaa atccctcaga aggagggata 4740

acaggatttg acctttacca gcgatttctg tccatatgtg gatgtaaaca gttctggaac 4800

gttatgcatg cagttagcga atccttgaat tatgttctgg tttgtacttg tcccatccat 4860

ccaaacaaga gattctgctt ttggtagcca tctgtagaaa catttaagat gtcactagaa 4920

tttacatttc atcctctcta cttgggttga ggttgcctat acttgcatat tgttaaaatg 4980

ttttggttgc tgatattcag aggaatgaaa cctggaacca aagcctaatt tgccgataaa 5040

aaaactgttt tcggccaggt gcagtggctc atgcctgtaa tcccagcacg ttgggaggcc 5100

gaggcgggtg gatcacctga agtcaggagt tcgagaccat cctggctaac actgtgaaac 5160

cccgtctcta ctaaaaatac aaaaaattag cggggcatgg tggcacgcgc ctgtagtccc 5220

agctactcag gaggctgagg caggagaatt acttgaaccc gggaggcgga ggttgcagta 5280

agcagagatt gtgccactgg actccagcct gggtgacaga gcgagactcc gtctcaaaca 5340

aacaaacaaa aaactgtttt catttgctct cttgaccaaa ggataggact ttagttcttt 5400

aagcattatt ttaaacacta tattgataca aaaatatctt gcttactcta aactttagag 5460

tctaaatgaa gctttttctc agtacaagat tctgagtatc ataaaatggt tatttaattg 5520

aaacgtagtg tggtatactc ttgatggtta gaactcttac agccttattt atttttaagt 5580

ttgttacagc caaagggttg gagtgtgcca gtgcacaggt agactaagga aaacattata 5640

gaggagtgaa gagaacagac cattgaaaag actattatct gaccagcgga ggcagaaaag 5700

agaggaaccc agttgaatag gatccaatcc ctggttagcc tctacacaat aatagggaga 5760

caaggattag gagccatacc tcccagagca aggtatcttt ctagagcaaa tttctctttc 5820

tagaagggga gggtcacagg gtcacagatt caccaaagct gaaagggctg aggagctcat 5880

ggtagcctgg gctgacctac tctggagcac ggtgtcttcc ttctaaactg agtgactgta 5940

gtactatctg tgcctctgat ggtaataaaa ctgacaagat gtctaatttt tttttaagta 6000

ggaccaaagg aaaacaagat ttagatagtc tgactttgct tttgaacaac agacattgca 6060

agtcaaaatt gttgtcaaat ttacatatgg taaatgatga actttaaaaa tgtgtccagg 6120

tgttagatga gttcattaga ctcttttaat gctaatggct agtacgttta aacaaaacag 6180

cagttctctg ctgcaatatt cccattgacc acttaaatga ccataagtgg tcatttaaga 6240

acatgttagg gttagccctg atctgaatat aaaagtgaga aaagggctac agtgcatttc 6300

ttggtaactt aaactgagtc ttgaagttat aatgatccat tcgagttctg tgatccttat 6360

tgttcttaat tgtgtttctc tacgtattgt tacagatgag ccatacgttt ctttgtatca 6420

atgtagacat gacttcagat acctctgagg acctacccag cagtctagga ccctgggcca 6480

agtgctggga ctatggtact aaatccagta gatgggctgt gtagcaactc tcccagggaa 6540

cacactaggg tacttaggga ggtgctttgt ggagcatgtt gaagctttga gatctgagca 6600

ggaggcagtg atgtccctgg tctattcagg gaaagatttc agtgtgaaat ggtaaacatc 6660

caattgacag gatttagatt ttgcttagtt tttctgcttt ttaatgtttc tatcccccat 6720

ctcagtgttt tctttatcca tcccagtgat gccttatttg aaactgggct taaactgcaa 6780

aaagaatgaa gttggattta ggaagctgtt agatcattga gtggtgttga gagtgaagtt 6840

tcactagcag ggaagtttcc ttgagcctaa aataaaaaga aaaaattaaa aagaatcagt 6900

ttttttaatt aaaaaaatag aaagctgtta ggctcctaat tcgtggggtt tttttttgta 6960

aaaacagttt agataatcct gaatgcaatc attaacttgg ttgctaatta caagaatgaa 7020

aattataatg gaaaaggaca aaataatata ccagctggtt tgttattata gtccgtgtat 7080

taaaatacta ttgaaatacg ttaaaggtaa atttttaagg tttaaaaaaa atttagtaac 7140

ttacagggat ggagaattta gatgtcagag gtggggagat ttatttttat aaggtaattt 7200

ttatcctgat aaggacttaa aaaaaagttt tgcaactgaa attttaaagt aaacatgtta 7260

agtacagtta aaaagtaagc attgtagtaa atagtggatt ctctggtgtg tattttttat 7320

ctcagtgttg aaaattggaa aagaatggac tgaagtctaa aaactggaat aatgaaggac 7380

actaaatgcc tttattgtag atactatgtt tgtaagtcta tagctaagca acttaagcca 7440

aaaaggtctt tcaactgaag ctttaatcaa cttattttgg agatgttctc ttcccttatc 7500

tcatgcgtca tccctaaaat aataagatac atgggatcaa atagcccttg ccttttcaac 7560

acaaatcagt tggaaaatta tggtttgagt cctgttgctg ccatggcttc tgtttctcag 7620

aaatgagtgt gtatgaacat accaatctat gtaataggct accttttttt gtcttctttg 7680

gaactttgta cacaaaccaa gacaatatca gggtgacagg tgaatgaact taaattctca 7740

gtcttgtcta ttcaccaaaa aagtatactg cctgtttttt ctttaattat tcaaggttga 7800

tgacttttag gaacatgttt tatactgtat tttttaatta aagcaagtgc cttgatgtaa 7860

ttccatgtaa atcattgctt aaccctctta tgggatgagg atgagttatt aatgtattgc 7920

agcctactgg aaaggagggg gagttggtta atagcagata cttttcttct agaagcttat 7980

gttttatgct gtttattatg taagatcctg tatgtgtgtt gagatttaga ggtttcattt 8040

gttttgtctg ctaataaatt gttactctaa taataaaaaa aa 8082

<210>  4
<211>  739
<212>  PRT
<213>  homo sapiens

<220>
<221>  PEPTIDE
<222>  (1)..(739)
<223>  Gene SLC26A2

<300>
<308>  NP_000103.2
<309>  2006-05-25
<313>  (1)..(739)

<400>  4

<210>  5
<211>  1103
<212>  DNA
<213>  Homo sapiens

<220>
<221>  misc_feature
<222>  (1)..(1103)
<223>  Gene GRO-1

<300>
<308>  NM_001511.1
<309>  1999-03-24
<313>  (1)..(1103)

<400>  5
cacagagccc gggccgcagg cacctcctcg ccagctcttc cgctcctctc acagccgcca      60

gacccgcctg ctgagcccca tggcccgcgc tgctctctcc gccgccccca gcaatccccg     120

gctcctgcga gtggcactgc tgctcctgct cctggtagcc gctggccggc gcgcagcagg     180

agcgtccgtg gccactgaac tgcgctgcca gtgcttgcag accctgcagg gaattcaccc     240

caagaacatc caaagtgtga acgtgaagtc ccccggaccc cactgcgccc aaaccgaagt     300

catagccaca ctcaagaatg ggcggaaagc ttgcctcaat cctgcatccc ccatagttaa     360

gaaaatcatc gaaaagatgc tgaacagtga caaatccaac tgaccagaag ggaggaggaa     420

gctcactggt ggctgttcct gaaggaggcc ctgcccttat aggaacagaa gaggaaagag     480

agacacagct gcagaggcca cctggattgt gcctaatgtg tttgagcatc gcttaggaga     540

agtcttctat ttatttattt attcattagt tttgaagatt ctatgttaat attttaggtg     600

taaaataatt aagggtatga ttaactctac ctgcacactg tcctattata ttcattcttt     660

ttgaaatgtc aaccccaagt tagttcaatc tggattcata tttaatttga aggtagaatg     720

ttttcaaatg ttctccagtc attatgttaa tatttctgag gagcctgcaa catgccagcc     780

actgtgatag aggctggcgg atccaagcaa atggccaatg agatcattgt gaaggcaggg     840

gaatgtatgt gcacatctgt tttgtaactg tttagatgaa tgtcagttgt tatttattga     900

aatgatttca cagtgtgtgg tcaacatttc tcatgttgaa actttaagaa ctaaaatgtt     960

ctaaatatcc cttggacatt ttatgtcttt cttgtaaggc atactgcctt gtttaatggt    1020

agttttacag tgtttctggc ttagaacaaa ggggcttaat tattgatgtt ttcatagaga    1080

atataaaaat aaagcactta tag                                            1103

<210>  6
<211>  107
<212>  PRT
<213>  Homo sapiens

<220>
<221>  protein
<222>  (1)..(107)
<223>  GRO-1

<300>
<308>  NP_001502.1
<309>  1999-03-24
<313>  (1)..(107)

<400>  6

Met Ala Arg Ala Ala Leu Ser Ala Ala Pro Ser Asn Pro Arg Leu Leu
1               5                   10                  15

Arg Val Ala Leu Leu Leu Leu Leu Leu Val Ala Ala Gly Arg Arg Ala
            20                  25                  30

Ala Gly Ala Ser Val Ala Thr Glu Leu Arg Cys Gln Cys Leu Gln Thr
        35                  40                  45

Leu Gln Gly Ile His Pro Lys Asn Ile Gln Ser Val Asn Val Lys Ser
    50                  55                  60

Pro Gly Pro His Cys Ala Gln Thr Glu Val Ile Ala Thr Leu Lys Asn
65                  70                  75                  80

Gly Arg Lys Ala Cys Leu Asn Pro Ala Ser Pro Ile Val Lys Lys Ile
                85                  90                  95

Ile Glu Lys Met Leu Asn Ser Asp Lys Ser Asn
            100                 105

<210>  7
<211>  1131
<212>  DNA
<213>  Homosapiens

<220>
<221>  gene
<222>  (1)..(1131)
<223>  Gene MMP-7

<300>
<308>  BC003635.2
<309>  2003-10-31
<313>  (1)..(1131)

<400>  7
   gtccaagaac aattgtctct ggacggcagc tatgcgactc accgtgctgt gtgctgtgtg 60
   cctgctgcct ggcagcctgg ccctgccgct gcctcaggag gcgggaggca tgagtgagct 120
   acagtgggaa caggctcagg actatctcaa gagattttat ctctatgact cagaaacaaa 180
   aaatgccaac agtttagaag ccaaactcaa ggagatgcaa aaattctttg gcctacctat 240
   aactggaatg ttaaactccc acgtcataga aataatgcag aagcccagat gtggagtgcc 300
   agatgttgca gaatactcac tatttccaaa tagcccaaaa tggacttcca aagtggtcac 360
   ctacaggatc gtatcatata ctcgagactt accgcatatt acagtggatc gattagtgtc 420
   aaaggcttta aacatgtggg gcaaagagat ccccctgcat ttcaggaaag ttgtatgggg 480
   aactgctgac atcatgattg gctttgcgcg aggagctcat ggggactcct acccatttga 540
   tgggccagga aacacgctgg ctcatgcctt tgcgcctggg acaggtctcg gaggagatgc 600
   tcacttcgat gaggatgaac gctggacgga tggtagcagt ctagggatta acttcctgta 660
   tgctgcaact catgaacttg gccattcttt gggtatggga cattcctctg atcctaatgc 720
   agtgatgtat ccaacctatg gaaatggaga tccccaaaat tttaaacttt cccaggatga 780
   tattaaaggc attcagaaac tatatggaaa gagaagtaat tcaagaaaga aatagaaact 840
   tcaggcagaa catccattca ttcattcatt ggattgtata tcattgttgc acaatcagaa 900
   ttgataagca ctgttcttcc actccattta gcaattatgt cacccttttt tattgcagtt 960
   ggtttttgaa tgtctttcac tccttttaag gataaactcc tttatggtgt gactgtgtct 1020
   tattcatcta tacttgcagt gggtagatgt caataaatgt tacatacaca aataaataaa 1080
   atgtttattc catggtaaat ttaaaaaaaa aaaaaaaaaa aaaaaaaaaa a 1131

<210> 8
<211> 267
<212> PRT
<213> Homo sapiens

<220>
<221> protein
<222> (1)..(267)
<223> MMP-7

<300>
<308> NP_002414.1
<309> 2003-10-31
<313> (1)..(267)

<400> 8

Met Arg Leu Thr Val Leu Cys Ala Val Cys Leu Leu Pro Gly Ser Leu
1               5                   10                  15

Ala Leu Pro Leu Pro Gln Glu Ala Gly Gly Met Ser Glu Leu Gln Trp
            20                  25                  30

Glu Gln Ala Gln Asp Tyr Leu Lys Arg Phe Tyr Leu Tyr Asp Ser Glu
        35                  40                  45

Thr Lys Asn Ala Asn Ser Leu Glu Ala Lys Leu Lys Glu Met Gln Lys
    50                  55                  60

Phe Phe Gly Leu Pro Ile Thr Gly Met Leu Asn Ser Arg Val Ile Glu
65                  70                  75                  80

Ile Met Gln Lys Pro Arg Cys Gly Val Pro Asp Val Ala Glu Tyr Ser
                85                  90                  95

Leu Phe Pro Asn Ser Pro Lys Trp Thr Ser Lys Val Val Thr Tyr Arg
            100                 105                 110

Ile Val Ser Tyr Thr Arg Asp Leu Pro His Ile Thr Val Asp Arg Leu
        115                 120                 125

Val Ser Lys Ala Leu Asn Met Trp Gly Lys Glu Ile Pro Leu His Phe
    130                 135                 140

Arg Lys Val Val Trp Gly Thr Ala Asp Ile Met Ile Gly Phe Ala Arg
145                 150                 155                 160

Gly Ala His Gly Asp Ser Tyr Pro Phe Asp Gly Pro Gly Asn Thr Leu
                165                 170                 175

Ala His Ala Phe Ala Pro Gly Thr Gly Leu Gly Gly Asp Ala His Phe
            180                 185                 190

Asp Glu Asp Glu Arg Trp Thr Asp Gly Ser Ser Leu Gly Ile Asn Phe
        195                 200                 205

Leu Tyr Ala Ala Thr His Glu Leu Gly His Ser Leu Gly Met Gly His
    210                 215                 220

Ser Ser Asp Pro Asn Ala Val Met Tyr Pro Thr Tyr Gly Asn Gly Asp
225                 230                 235                 240

Pro Gln Asn Phe Lys Leu Ser Gln Asp Asp Ile Lys Gly Ile Gln Lys
                245                 250                 255

Leu Tyr Gly Lys Arg Ser Asn Ser Arg Lys Lys
            260                 265

<210>  9
<211>  894
<212>  DNA
<213>  Homo sapiens

<220>
<221>  Gene
<222>  (1)..(894)
<223>  Gene MAP-17

<300>
<308>  NM_005764.3
<309>  2004-01-23
<313>  (1)..(894)

<400>  9
gcccgtcttc gtgtctcctc cctccctcgc cttcctcctt cctagctcct ctcctccagg      60

gccagactga gcccaggttg atttcaggcg gacaccaata gactccacag cagctccagg     120

agcccagaca ccggcggcca gaagcaaggc taggagctgc tgcagccatg tcggccctca     180

gcctcctcat tctgggcctg ctcacggcag tgccacctgc cagctgtcag caaggcctgg     240

ggaaccttca gccctggatg cagggcctta tcgcggtggc cgtgttcctg gtcctcgttg     300

caatcgcctt tgcagtcaac cacttctggt gccaggagga gccggagcct gcacacatga     360

tcctgaccgt cggaaacaag gcagatggag tcctggtggg aacagatgga aggtactctt     420

cgatggcggc cagtttcagg tccagtgagc atgagaatgc ctatgagaat gtgcccgagg     480

aggaaggcaa ggtccgcagc accccgatgt aaccttctct gtggctccaa ccccaagact     540

cccaggcaca tgggatggat gtccagtgct accacccaag ccccctcctt ctttgtgtgg     600

aatctgcaat agtgggctga ctccctccag ccccatgccg gccctacccg cccttgaagt     660

atagccagcc aaggttggag ctcagaccgt gtctaggttg gggctcggct gtggccctgg     720

ggtctcctgc tcagctcaga agagccttct ggagaggaca gtcagctgag cacctcccat     780

cctgctcaca cgtccttccc cataactatg gaaatggccc taatttctgt gaaataaaga     840

ctttttgtat ttctggggct gaggctcagc aacagcccct caggcttcca gtga           894


<210>  10
<211>  114
<212>  PRT
<213>  Homo sapiens


<220>
<221>  Protein
<222>  (1)..(114)
<223>  MAP-17

<300>
<308>  NP_005755.1
<309>  2004-01-23
<313>  (1)..(114)

<400>  10

Met Ser Ala Leu Ser Leu Leu Ile Leu Gly Leu Leu Thr Ala Val Pro
1               5                   10                  15

Pro Ala Ser Cys Gln Gln Gly Leu Gly Asn Leu Gln Pro Trp Met Gln
            20                  25                  30

Gly Leu Ile Ala Val Ala Val Phe Leu Val Leu Val Ala Ile Ala Phe
        35                  40                  45

Ala Val Asn His Phe Trp Cys Gln Glu Glu Pro Glu Pro Ala His Met
    50                  55                  60

Ile Leu Thr Val Gly Asn Lys Ala Asp Gly Val Leu Val Gly Thr Asp
65                  70                  75                  80

Gly Arg Tyr Ser Ser Met Ala Ala Ser Phe Arg Ser Ser Glu His Glu
                85                  90                  95

Asn Ala Tyr Glu Asn Val Pro Glu Glu Glu Gly Lys Val Arg Ser Thr
            100                 105                 110

Pro Met

<210>  11
<211>  1180
<212>  DNA
<213>  Homo sapiens

<220>
<221>  Gene
<222>  (1)..(1180)
<223>  Gene RegIV

<300>
<308>  BC17089.2
<309>  2003-08-25
<313>  (1)..(1180)

<400>  11
cactgaagaa ggcaggggcc cttagagtct tggttgccaa acagatttgc agatcaagga      60

gaacccagga gtttcaaaga agcgctagta aggtctctga gatccttgca ctagctacat     120

cctcagggta ggaggaagat ggcttccaga agcatgcggc tgctcctatt gctgagctgc     180

ctggccaaaa caggagtcct gggtgatatc atcatgagac ccagctgtgc tcctggatgg     240

ttttaccaca agtccaattg ctatggttac ttcaggaagc tgaggaactg gtctgatgcc     300

gagctcgagt gtcagtctta cggaaacgga gcccacctgg catctatcct gagtttaaag     360

gaagccagca ccatagcaga gtacataagt ggctatcaga gaagccagcc gatatggatt     420

ggcctgcacg acccacagaa gaggcagcag tggcagtgga ttgatggggc catgtatctg     480

tacagatcct ggtctggcaa gtccatgggt gggaacaagc actgtgctga gatgagctcc     540

aataacaact ttttaacttg gagcagcaac gaatgcaaca agcgccaaca cttcctgtgc     600

aagtaccgac catagagcaa gaatcaagat tctgctaact cctgcacagc cccgtcctct 660

tcctttctgc tagcctggct aaatctgctc attatttcag aggggaaacc tagcaaacta 720

agagtgataa gggccctact acactggctt ttttaggctt agagacagaa actttagcat 780

tggcccagta gtggcttcta gctctaaatg tttgccccgc catccctttc cacagtatcc 840

ttcttccctc ctcccctgtc tctggctgtc tcgagcagtc tagaagagtg catctccagc 900

ctatgaaaca gctgggtctt tggccataag aagtaaagat ttgaagacag aaggaagaaa 960

ctcaggagta agcttctagc ccccttcagc ttctacaccc ttctgccctc tctccattgc 1020

ctgcacccca ccccagccac tcaactcctg cttgtttttc ctttggccat gggaaggttt 1080

accagtagaa tccttgctag gttgatgtgg gccatacatt cctttaataa accattgtgt 1140

acataaaaaa aaaaaaaaaa aaaaaaaaaa aaaaaaaaaa 1180

<210> 12
<211> 158
<212> PRT
<213> Homo sapiens

<220>
<221> Protein
<222> (1)..(158)
<223> RegIV

<300>
<308> NP_114433.1
<309> 2001-05-15
<313> (1)..(158)

<400> 12

Met Ala Ser Arg Ser Met Arg Leu Leu Leu Leu Leu Ser Cys Leu Ala
1               5                   10                  15

Lys Thr Gly Val Leu Gly Asp Ile Ile Met Arg Pro Ser Cys Ala Pro
            20                  25                  30

Gly Trp Phe Tyr His Lys Ser Asn Cys Tyr Gly Tyr Phe Arg Lys Leu
        35                  40                  45

Arg Asn Trp Ser Asp Ala Glu Leu Glu Cys Gln Ser Tyr Gly Asn Gly
    50                  55                  60

Ala His Leu Ala Ser Ile Leu Ser Leu Lys Glu Ala Ser Thr Ile Ala
65                  70                  75                  80

Glu Tyr Ile Ser Gly Tyr Gln Arg Ser Gln Pro Ile Trp Ile Gly Leu
                85                  90                  95

His Asp Pro Gln Lys Arg Gln Gln Trp Gln Trp Ile Asp Gly Ala Met
            100                 105                 110

Tyr Leu Tyr Arg Ser Trp Ser Gly Lys Ser Met Gly Gly Asn Lys His
        115                 120                 125

Cys Ala Glu Met Ser Ser Asn Asn Asn Phe Leu Thr Trp Ser Ser Asn
    130                 135                 140

Glu Cys Asn Lys Arg Gln His Phe Leu Cys Lys Tyr Arg Pro
145                 150                 155

<210>  13
<211>  3109
<212>  DNA
<213>  Homo sapiens

<220>
<221>  Gene
<222>  (1)..(3109)
<223>  Gene Vanin-1

<300>
<308>  NM_004666.1
<309>  1999-05-07
<313>  (1)..(3109)

<400>  13
cattggactt cagcatgact actcagttgc cagcttacgt ggcaattttg cttttctatg      60

tctcaagagc cagctgccag gacactttca ttgcagctgt ttatgagcat gcagcgatat     120

tgcccaatgc caccctaaca ccagtgtctc gtgaggaggc tttggcatta atgaatcgga     180

atctggacat tttggaagga gcgatcacat cagcagcaga tcagggtgcg catattattg     240

tgactccaga agatgctatt tatggctgga acttcaacag ggactctctc tacccatatt     300

tggaggacat cccagaccct gaagtaaact ggatcccctg taataatcgt aacagatttg     360

gccagacccc agtacaagaa agactcagct gcctggccaa gaacaactct atctatgttg     420

tggcaaatat tggggacaag aagccatgcg ataccagtga tcctcagtgt ccccctgatg     480

gccgttacca atacaacact gatgtggtat ttgattctca aggaaaactg gtggcacgct     540

accataagca aaaccttttc atgggtgaaa atcaattcaa tgtacccaag gagcctgaga     600

ttgtgacttt caataccacc tttggaagtt ttggcatttt cacatgcttt gatatactct     660

tccatgatcc tgctgttacc ttggtgaaag atttccacgt ggacaccata gtattcccaa     720

cagcttggat gaatgttttg ccacatttgt cagctgttga attccactca gcttgggcta     780

tgggcatgag ggtcaatttc cttgcatcca acatacatta cccctcaaag aaaatgacag     840

gaagtggcat ctatgcaccc aattcttcaa gagcatttca ttatgatatg aagacagaag     900

agggaaaact cctcctctcg caactggatt cccacccatc ccattctgca gtggtgaact     960

ggacttccta tgccagcagt atagaagcgc tctcatcagg aaacaaggaa tttaaaggca    1020

ctgtcttttt cgatgaattc acttttgtga agctcacagg agttgcagga aattatacag 1080

tttgtcagaa agatctctgc tgtcatttaa gctacaaaat gtctgagaac ataccaaatg 1140

aagtgtacgc tctaggggca tttgacggac tgcacactgt ggaagggcgc tattatctac 1200

agatttgtac cctgttgaaa tgtaaaacga ctaatttaaa cacttgcggt gactcagctg 1260

aaacagcttc taccaggttt gaaatgttct ccctcagtgg cactttcgga acccagtatg 1320

tctttcctga ggtgttgctg agtgaaaatc agcttgcacc tggagaattt caggtgtcaa 1380

ctgacggacg cttgtttagt ctgaagccaa catccggacc tgtcttaaca gtaactctgt 1440

ttgggaggtt gtatgagaag gactgggcat caaatgcttc atcaggcctc acagcacaag 1500

caagaataat aatgctaata gttatagcac ctattgtatg ctcattaagt tggtagaata 1560

ttgacttttt ctctttttta tttgggataa tttaaaaaat gatggatgag aaaagaaaga 1620

ttggtccggg ttaatattat cctctagtat aagtgaatta ctagtttctc tttatttaga 1680

caaacacaca cacaccagat aatataaact taataaatta tctgttaatg tagattttat 1740

ttaaaaaact atatttgaac attggtcttt cttggacgtg agctaattat atcaaataag 1800

tatcacaaat cttttacgca gaagaaataa aaactacggg tagaaaacat aagaactatc 1860

ataaaattta cttacaagga ggctgctctt gttaccactt ttattatatt acgtatcact 1920

tattcagctc tgctgaaaat ttccaatgac tttgtttgtt tgctctttta gttttttacc 1980

taaacaatac attttgattc tcttgtgggt tgataatgtc tccccaaaat ttacatgttg 2040

aagcacctca gaatgtgact gtatttggag acagggtctt taaagaggta aaataaggtc 2100

attaggatag accctaattc aatatgactg atgatcataa aagaagaggc gagtagggca 2160

caacaggcac aaagggagac cataaggaga cacagaggaa ggacaactct ttacaagcta 2220

agaagagagg gcctcagaag aaaccaaccc tgccaacacc ttgatcttgg acttccagcc 2280

tccaaaacta tgagaaataa atttctattg tttaagtcac ccagtccatg gtactttgtt 2340

aggcagccct ggcaaatgaa tcaaagaccc attcctgttc ctctccccac cactactgtt 2400

ttctactgta atctgaagct tcaacaaaag gcttacctgg taagaatatt cagctggtct 2460

gggtcctcaa gactccaata gacactctta aagaaggatt gctgatggat tgatagtgaa 2520

accattagat cattgaattc ctctggaatt agaaaaccag agagtcccat tttaagaaat 2580

tagatattta atatagcatt gtgtgttcta ttttagtaac agcagaatct cttgacatta 2640

cacaactcag tgaaacaaca tcatttaagc caaaatatct cccaactgac tgatagactc 2700

tgagcactaa tatcatagtg ctgtgatgat ggacaattac atagtaccga taacagccat 2760

gcactgtgca aagcatgccc ttctgcacag gagagcaagg cacttgcagt agtgatctat 2820

gccagcaaaa catcattttg agacaaacat ttttgtggca gatgtttttc ctaaaaagta 2880

ctatatcatc caagaaatat ttgagtaaaa tcccttgttc ttttgggtga cattaactga 2940

catttgcttt ttttcaagac ctaatagaaa ataagaaagc ccataatgta tttagaaaca 3000

ggaatcctca gagcaattct ctgtattctc atataatttc aatgtaaaac agaaaacata 3060

ttgatgtgtt ggtgataggc ttgaattatt aaaaacttca aaaacaaaa 3109

<210> 14
<211> 513
<212> PRT
<213> Homo sapiens

<220>
<221> Protein
<222> (1)..(513)
<223> Vanin-1

<300>
<308> NP_004657.1
<309> 1999-05-07
<313> (1)..(513)

<400> 14

Met Thr Thr Gln Leu Pro Ala Tyr Val Ala Ile Leu Leu Phe Tyr Val
1               5                   10                  15

Ser Arg Ala Ser Cys Gln Asp Thr Phe Ile Ala Ala Val Tyr Glu His
            20                  25                  30

Ala Ala Ile Leu Pro Asn Ala Thr Leu Thr Pro Val Ser Arg Glu Glu
        35                  40                  45

Ala Leu Ala Leu Met Asn Arg Asn Leu Asp Ile Leu Glu Gly Ala Ile
    50                  55                  60

Thr Ser Ala Ala Asp Gln Gly Ala His Ile Ile Val Thr Pro Glu Asp
65                  70                  75                  80

Ala Ile Tyr Gly Trp Asn Phe Asn Arg Asp Ser Leu Tyr Pro Tyr Leu
                85                  90                  95

Glu Asp Ile Pro Asp Pro Glu Val Asn Trp Ile Pro Cys Asn Asn Arg
            100                 105                 110

Asn Arg Phe Gly Gln Thr Pro Val Gln Glu Arg Leu Ser Cys Leu Ala
        115                 120                 125

Lys Asn Asn Ser Ile Tyr Val Val Ala Asn Ile Gly Asp Lys Lys Pro
    130                 135                 140

Cys Asp Thr Ser Asp Pro Gln Cys Pro Pro Asp Gly Arg Tyr Gln Tyr
145                 150                 155                 160

Asn Thr Asp Val Val Phe Asp Ser Gln Gly Lys Leu Val Ala Arg Tyr

165                 170                 175

His Lys Gln Asn Leu Phe Met Gly Glu Asn Gln Phe Asn Val Pro Lys
            180                 185                 190

Glu Pro Glu Ile Val Thr Phe Asn Thr Thr Phe Gly Ser Phe Gly Ile
        195                 200                 205

Phe Thr Cys Phe Asp Ile Leu Phe His Asp Pro Ala Val Thr Leu Val
    210                 215                 220

Lys Asp Phe His Val Asp Thr Ile Val Phe Pro Thr Ala Trp Met Asn
225                 230                 235                 240

Val Leu Pro His Leu Ser Ala Val Glu Phe His Ser Ala Trp Ala Met
                245                 250                 255

Gly Met Arg Val Asn Phe Leu Ala Ser Asn Ile His Tyr Pro Ser Lys
            260                 265                 270

Lys Met Thr Gly Ser Gly Ile Tyr Ala Pro Asn Ser Ser Arg Ala Phe
        275                 280                 285

His Tyr Asp Met Lys Thr Glu Glu Gly Lys Leu Leu Leu Ser Gln Leu
    290                 295                 300

Asp Ser His Pro Ser His Ser Ala Val Val Asn Trp Thr Ser Tyr Ala
305                 310                 315                 320

Ser Ser Ile Glu Ala Leu Ser Ser Gly Asn Lys Glu Phe Lys Gly Thr
                325                 330                 335

Val Phe Phe Asp Glu Phe Thr Phe Val Lys Leu Thr Gly Val Ala Gly
            340                 345                 350

Asn Tyr Thr Val Cys Gln Lys Asp Leu Cys Cys His Leu Ser Tyr Lys
        355                 360                 365

Met Ser Glu Asn Ile Pro Asn Glu Val Tyr Ala Leu Gly Ala Phe Asp
    370                 375                 380

Gly Leu His Thr Val Glu Gly Arg Tyr Tyr Leu Gln Ile Cys Thr Leu
385                 390                 395                 400

Leu Lys Cys Lys Thr Thr Asn Leu Asn Thr Cys Gly Asp Ser Ala Glu
                405                 410                 415

Thr Ala Ser Thr Arg Phe Glu Met Phe Ser Leu Ser Gly Thr Phe Gly
            420                 425                 430

Thr Gln Tyr Val Phe Pro Glu Val Leu Leu Ser Glu Asn Gln Leu Ala
        435                 440                 445

Pro Gly Glu Phe Gln Val Ser Thr Asp Gly Arg Leu Phe Ser Leu Lys
    450                 455                 460

Pro Thr Ser Gly Pro Val Leu Thr Val Thr Leu Phe Gly Arg Leu Tyr
465                 470                 475                 480

Glu Lys Asp Trp Ala Ser Asn Ala Ser Ser Gly Leu Thr Ala Gln Ala
                485                 490                 495

Arg Ile Ile Met Leu Ile Val Ile Ala Pro Ile Val Cys Ser Leu Ser
            500                 505                 510

Trp

<210>  15
<211>  22
<212>  DNA
<213>  Artificial Sequence

<220>
<223>  Oligo-dT-primer

<220>
<221>  misc_feature
<222>  (1)..(20)

<220>
<221>  misc_feature
<222>  (21)..(21)
<223>  A, T, C or G

<220>
<221>  misc_feature
<222>  (22)..(22)
<223>  A, G or C

<400>  15
tttttttttt tttttttttt nv                                                22

<210>  16
<211>  24
<212>  DNA
<213>  Artificial Sequence

<220>
<223>  primer

<220>
<221>  misc_feature
<222>  (1)..(24)

<223>  Forward primer for SLCA14

<400>  16
gagcaaagag gtggatattc tggc                                              24


<210>  17
<211>  24
<212>  DNA
<213>  Artificial Sequence

<220>
<223>  primer


<220>
<221>  misc_feature
<222>  (1)..(24)
<223>  Reverse primer for SLC6A14

<400>  17
ctccccagtc agggtatgga attg                                              24


<210>  18
<211>  24
<212>  DNA
<213>  Artificial Sequence

<220>
<223>  primer


<220>
<221>  misc_feature
<222>  (1)..(24)
<223>  Forward primer for SLC26A2

<400>  18
cacctaaagc tattatgcag gagg                                              24


<210>  19
<211>  24
<212>  DNA
<213>  Artificial Sequence

<220>
<223>  primer


<220>
<221>  misc_feature
<222>  (1)..(24)
<223>  Reverse primer SLC26A2

<400>  19
ctcctcaatt catgacctgt gggc                                              24


<210>  20
<211>  24
<212>  DNA
<213>  Artificial Sequence

<220>
<223>  primer


<220>
<221>  misc_feature
<222>  (1)..(24)
<223>  Forward primer for GRO-1

<400>  20
gccaatgaga tcattgtgaa ggca                                              24


<210>  21
<211>  24
<212>  DNA
<213>  Artificial Sequence

<220>
<223>  primer


<220>
<221>  misc_feature
<222>  (1)..(24)
<223>  reverse primer for GRO-1

<400>  21
caacatgaga aatgttgacc acac                                              24


<210>  22
<211>  24
<212>  DNA
<213>  Artificial Sequence

<220>
<223>  primer


<220>
<221>  misc_feature
<222>  (1)..(24)
<223>  Forward primer for MMP-7

<400>  22
cactgttctt ccactccatt tagc                                              24


<210>  23
<211>  24
<212>  DNA
<213>  Artificial Sequence

<220>
<223>  primer


<220>
<221>  misc_feature
<222>  (1)..(24)
<223>  Reverse primer for MMP-7

<400>  23
gacatctacc cactgcaagt atag                                              24

<210>  24
<211>  24
<212>  DNA
<213>  Artificial Sequence

<220>
<223>  primer'


<220>
<221>  misc_feature
<222>  (1)..(24)
<223>  Reverse primer for MAP-17

<400>  24
gttcctggtc ctcgttgcaa tcgc                                              24


<210>  25
<211>  24
<212>  DNA
<213>  Artificial Sequence

<220>
<223>  primer


<220>
<221>  misc_feature
<222>  (1)..(24)
<223>  Reverse primer for MAP-17

<400>  25
ccatcgaaga gtaccttcca tctg                                              24


<210>  26
<211>  24
<212>  DNA
<213>  Artificial Sequence

<220>
<223>  primer


<220>
<221>  misc_feature
<222>  (1)..(24)
<223>  Forward primer for RegIV

<400>  26
ggtgatatca tcatgagacc cagc                                              24


<210>  27
<211>  24
<212>  DNA
<213>  Artificial Sequence

<220>
<223>  primer

<220>
<221>  misc_feature
<222>  (1)..(24)
<223>  Reverse primer for RegIV

<400>  27
ctttaaactc aggatagatg ccag                                              24


<210>  28
<211>  24
<212>  DNA
<213>  Artificial Sequence

<220>
<223>  primer


<220>
<221>  misc_feature
<222>  (1)..(24)
<223>  Forward primer for Vanin-1

<400>  28
ccaactgact gatagactct gagc                                              24


<210>  29
<211>  24
<212>  DNA
<213>  Artificial Sequence

<220>
<223>  primer


<220>
<221>  misc_feature
<222>  (1)..(24)
<223>  Reverse primer for Vanin-1

<400>  29
ggcatagatc actactgcaa gtgc                                              24


<210>  30
<211>  24
<212>  DNA
<213>  Artificial Sequence

<220>
<223>  primer


<220>
<221>  misc_feature
<222>  (1)..(24)
<223>  Forward primer for gamma actin

<400>  30
gtgcagggta ttaacgtgtc aggg                                              24


<210>  31
<211>  28

<212>  DNA
<213>  Artificial Sequence

<220>
<223>  primer



<220>
<221>  misc_feature
<222>  (1)..(28)
<223>  Reverse primer for gamma actin

<400>  31
ccaactcaaa gcaagtaaca gcccacgg                                          28